# EUROPEAN PATENT APPLICATION

(11) **EP 4 293 066 A1**
(43) Date of publication of application: **20.12.2023**
(21) Application number: 22752644.9
(22) Date of filing: 02.02.2022
(51) Int. Cl.: C08G 77/14, A61Q 17/04, C08L 83/05, C08L 83/07, A61K 8/893

(54) **ORGANOPOLYSILOXANE AND COSMETIC CONTAINING SAME**

(30) Priority: 10.02.2021 JP 2021019864
(71) Applicant: SHIN-ETSU CHEMICAL CO., LTD., Tokyo 1000005 (JP)
(72) Inventor: IMAI Taro, Annaka-shi, Gunma 379-0224 (JP); MIYAUCHI Masaru, Tokyo 100-0005 (JP); KONISHI Masayuki, Tokyo 100-0005 (JP)
(74) Representative: Mewburn Ellis LLP
(86) International application number: PCT/JP2022/003918
(87) International publication number: WO 2022/172816

(57) **Abstract**

Provided is an alkyl-glycerin co-modified branched organopolysiloxane (polyglycerin-modified silicone in which a silicone chain and an alkyl chain are branched at a silicone main chain) having long-chain alkyl groups, glycerin groups, and branched-chain silicone groups, wherein: the organopolysiloxane is an organosiloxane in which the proportions of siloxane units having long-chain alkyl groups, siloxane units having glycerin groups, and siloxane units having branched-chain silicone groups, the chain length of the organopolysiloxane, the ratio of siloxane units, the amount of long-chain alkyl groups, and the amount of glycerin groups are specified; when the organopolysiloxane is blended with a composition containing a powder, the dispersibility and dispersion stability are excellent; and when the organopolysiloxane is blended with a cosmetic, a cosmetic having excellent spreadability and blendability with the skin as well as exceptional stability over time is obtained even when the cosmetic contains an oil-soluble UV absorber.

## Description

### TECHNICAL FIELD

This invention relates to an alkyl/glycerin-co-modified branched organopolysiloxane and a cosmetic composition comprising the organopolysiloxane. Herein, cosmetic compositions are also referred to as cosmetics. The name of ingredients is sometimes expressed by the International Nomenclature for Cosmetic Ingredients (INCI).

### BACKGROUND ART

Glycerin or polyglycerin-modified silicone base surfactants are often used in cosmetics because of their moisture retention, but are known to have a tendency to functionally turn sticky (Patent Document 1).

On the other hand, powders including inorganic powders, organic powders, and coloring matter powders are formulated in cosmetics. They exert a make-up effect and feel-improving effect. In particular, metal oxides such as titanium oxide and zinc oxide are used not only generally as white pigments capable of hiding skin troubles, but also frequently as UV protecting agents having chemical and physical stability and high safety.

However, it is generally difficult to disperse microparticulate powders such as metal oxides in cosmetics in a stable manner to a high concentration. Agglomeration occurs due to the electric charges and polarity the powder possesses and minute amounts of impurities in the powder, detracting from dispersibility and stability. For example, a cosmetic composition containing a pigment in the form of microscopic particles manifests an outstanding viscosity buildup with the lapse of time. The cosmetic composition is not only susceptible to make-up degradation, but also gels and becomes difficult to apply.

Thus, for the purposes of preventing a microparticulate powder in a cosmetic composition from agglomerating, improving its dispersion and stability, and ameliorating the feeling of the cosmetic composition, some techniques have hitherto been proposed, for example, the technique of treating powders on their surface with various surface treating agents and the technique of adding dispersants to powders. In particular, since many cosmetics have an oil and a silicone oil blended therein from the aspects of feeling, water repellency, and safety, a number of techniques have been developed relating to powder dispersants using modified silicones which are fully compatible with silicone oil. For example, Patent Document 2 proposes surface modification with straight, single end alkoxy-modified silicone, Patent Document 3 proposes the use of polyether-modified silicone as the dispersant, and Patent Document 4 proposes the use of polyglycerin-modified silicone.

Although the techniques using such modified silicones are effective for preventing or improving the agglomeration and settlement of particles, the extent of improvement is still insufficient. Particularly in cosmetics, this raises such problems as color darkening or a loss of transparency due to agglomeration of particles, emphasis of unnatural whiteness, aggravation of spread on the skin, and degraded feeling on use.

Moreover, as the recent enlargement of the sun screen market entails the demand for cosmetics capable of exerting more UV-protecting effect than in the prior art, the method of increasing the content of metal oxides in cosmetics and the method of using organic UV-absorbers in combination are employed. However, the prior art modified silicones mentioned above experience a large viscosity buildup with the lapse of time in case of a high concentration of powder. Particularly when organic UV absorbers are used in combination, dispersion stability with time is not maintained due to poor compatibility between the silicone oil and modified silicone and the organic UV absorber having a high polarity, raising the problem of an outstanding viscosity buildup of the dispersion.

It is then contemplated to solve the problem by using not only the silicone oil as the oil, but also a compatibilizing agent such as ester oil or long-chain alkyl-modified silicone in combination, for thereby improving the compatibility between the oil and the organic UV absorber. This approach rather increases the polarity of the overall oil to aggravate the compatibility between the polyether-modified silicone or polyglycerin-modified silicone and the mixing oil, failing to provide a stable dispersion.

On the other hand, as the modified silicone which is compatible with highly polar oils, Patent Document 5 proposes a silicone compound having both a long chain alkyl group and a polyoxyalkylene group compatible with ester oils and hydrocarbon oils; and Patent Document 6 proposes a silicone compound having both a long-chain alkyl group and a polyhydric alcohol. However, although these silicone compounds display a high emulsifying power in mixed oil systems, they lack a sufficient ability in the dispersing force aspect of dispersing a powder such as metal oxide in an oil. The structure suited for that purpose is discussed nowhere. In addition, although the emulsified systems containing the modified silicones disclosed in Patent Documents 5 and 6 in combination with ester oils or hydrocarbon oils are improved in emulsion stability, problems arise that a heavy feeling derived from the intermolecular force between long-chain alkyl group and the oil manifests strongly to give a sticky impression particularly when the ester oils and hydrocarbon oils are non-volatile oils. It is desired to further improve the feeling, and to have an emulsifier capable of providing a light feeling particularly when the ester oils or hydrocarbon oils are used. Also, although the above-mentioned silicone compounds exert high compatibility in mixed oil systems, they fail to prevent agglomeration of metal oxides or the like or entail an outstanding viscosity buildup, when used for the purpose of dispersing metal oxides or the like in single silicone oil systems.

As discussed above, it is desired to develop a surface treating agent/dispersing agent capable of reducing the stickiness inherent to glycerin-modified silicone, providing a dispersion having a low viscosity and high age stability even when a single silicone oil system is heavily loaded with a powder such as metal oxide, preventing agglomeration of powder and dispersing to a full extent even in a mixed oil system where an organic UV absorber is used in combination with a metal oxide.

### PRIOR ART DOCUMENTS

### PATENT DOCUMENTS

Patent Document 1: JP-A 2002-003334
Patent Document 2: JP-A H07-196946
Patent Document 3: JP-A H10-167946
Patent Document 4: JP-A H10-316536
Patent Document 5: JP-A S61-090732
Patent Document 6: JP-A 2002-179798

### SUMMARY OF INVENTION

### TECHNICAL PROBLEM

An object of the invention, which has been made under the above-mentioned circumstances, is to provide an organopolysiloxane which exhibits satisfactory dispersibility and dispersion stability when blended in a powder-containing composition, and which offers a cosmetic composition having a high age stability and satisfactory spreadability and skin-adaptability when blended in a cosmetic composition, despite of containment of an oil-soluble UV absorber, and a cosmetic composition comprising the same.

### SOLUTION TO PROBLEM

Making extensive investigations to attain the above object, the inventors have found that an alkyl/glycerin-co-modified branched organopolysiloxane having a long-chain alkyl group, a glycerin group, and a branched silicone group (i.e., polyglycerin-modified silicone having a silicone chain and an alkyl chain branching from the silicone backbone) is highly compatible with a wide range of oils encompassing silicone oils, ester oils, and hydrocarbon oils, when the proportions of siloxane units having a long-chain alkyl group, siloxane units having a glycerin group, and siloxane units having a branched silicone group are specified, the chain length of the organopolysiloxane is specified, the ratio of siloxane units is specified, the content of the long-chain alkyl group is specified, and the content of the glycerin group is specified. In particular, the organopolysiloxane is able to disperse a powder in an oil in a high concentration when its viscosity is in a range of 200 to 3,000 mPa-s, solving the outstanding problems. The invention is predicated on this finding.

Accordingly, the invention provides an organopolysiloxane and a cosmetic composition comprising the same, as defined below.
1. An organopolysiloxane having the compositional formula (1):
   [Chem. 1]

   (R¹₂SiO_{2/2})ₐ(R¹R²SiO_{2/2})_{b}(R¹R³SiO_{2/2})_{c}(R¹R⁴SiO_{2/2})_{d}(R¹SiO_{3/2})ₑ(SiO_{4/2})_{f}(R¹₃SiO_{1/2})_{2+e+2f} (1)

   wherein R¹ is independently a monovalent hydrocarbon group selected from C₁-C₅ alkyl groups, C₆-C₁₅ aryl groups, and C₇-C₁₅ aralkyl groups,
      R² is a C₆-C₂₀ alkyl group,
      R³ is a group having the formula (3) which is bonded to a silicon atom in the polysiloxane backbone via a linking group having the formula (2):

         -Q-O- (2)
   wherein Q is a C₂-C₂₀ divalent hydrocarbon group,
   or a glycerin group consisting of 2 to 10 glycerol units selected from the formulae (3) to (9): 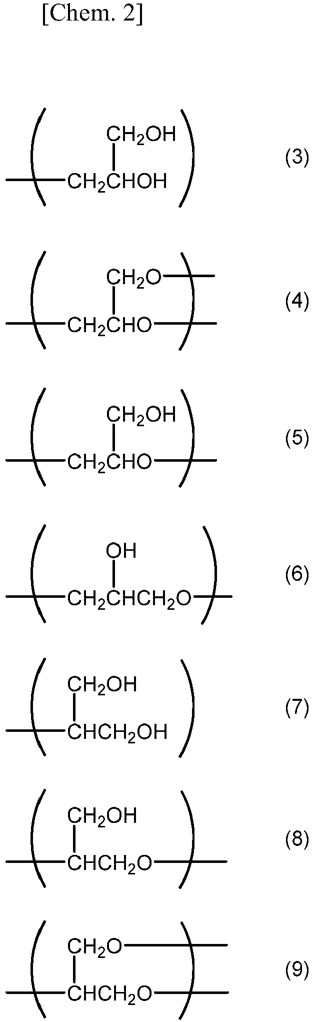
   which is bonded to a silicon atom in the polysiloxane backbone via a linking group having the formula (2),
      R⁴ is a group having the general formula (10), (11), (12) or (13):
      [Chem. 3]

      -(CH₂)₂-C_{g}H_{2g}-(SiOR¹₂)ₕ-SiR¹₃ (10)

      -(CH₂)₂-C_{g}H_{2g}-SiR¹ₕ₁-(OSiR¹₃)₃₋ₕ₁ (11)

      -(CH₂)₂-C_{g}H_{2g}-SiR¹ₕ₁-(OSiR¹ₕ₂(OSiR¹₃)₃₋ₕ₂)₃₋ₕ₁ (12)

      -(CH₂)₂-C_{g}H_{2g}-SiR¹ₕ₁-(OSiR¹h₂(OSiR1h₃(OSiR13)₃.ₕ₃)₃₋ₕ₂)₃₋ₕᵢ (13)
   wherein R¹ is as defined above, g and h each are an integer in the range: 0 ≤ g ≤ 5 and
      0 ≤ h ≤ 100, h1 to h3 each are an integer of 0 to 2,
      a, b, c, d, e, and f each are a number in the range: 1 ≤ a ≤ 9, 3 < b ≤ 8, 0.4 ≤ c ≤ 3, 0.3 ≤ d ≤ 5, e ≥ 0, and f ≥ 0, 7 ≤ a+b+c+d ≤ 20, and 0.2 ≤ (b+c)/(a+d) ≤ 3,
      the organopolysiloxane having a viscosity of 200 to 3,000 mPa·s as measured at 25°C by a B-type viscometer according to the method of JIS K 7117-1: 1999.
2. The organopolysiloxane of 1 wherein the total amount of R² is 20 to 40% by weight of one molecule, and the total amount of R³ is 5 to 20% by weight of one molecule.
3. The organopolysiloxane of 1 or 2, having a weight average molecular weight of 1,500 to 7,500 as measured by gel permeation chromatography versus polystyrene standards.
4. The organopolysiloxane of any one of 1 to 3 wherein R² in formula (1) is a C₁₂ alkyl group.
5. The organopolysiloxane of any one of 1 to 4 wherein R³ in formula (1) is selected from groups having the general formulae (14) to (20). 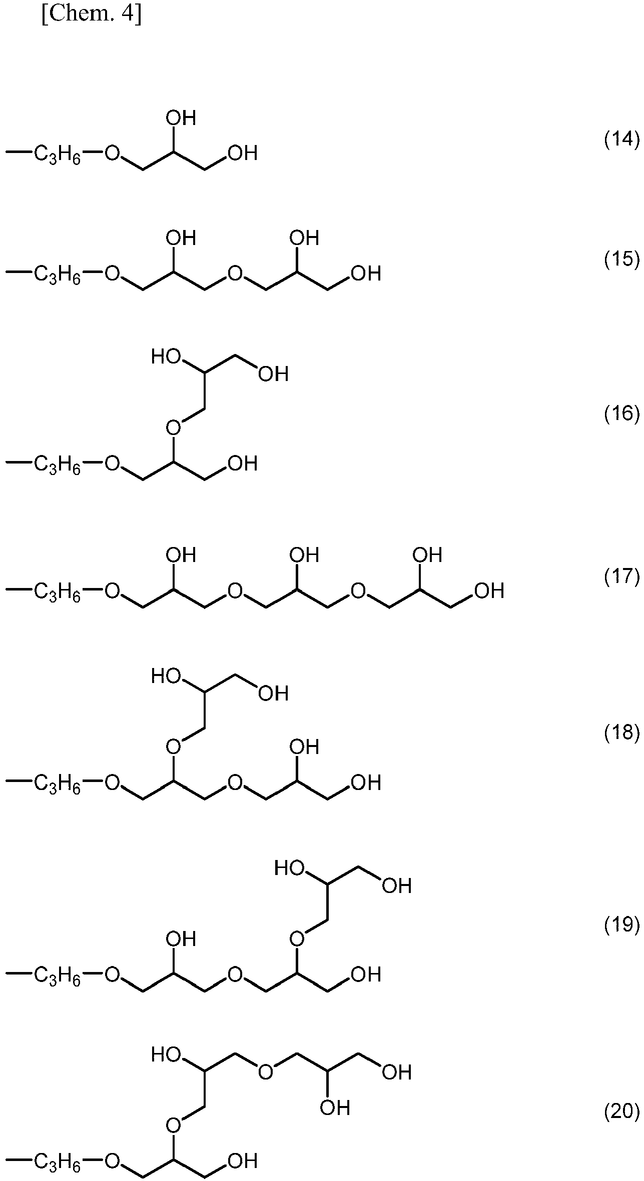
6. A cosmetic composition comprising (A) the organopolysiloxane of any one of 1 to 5.
7. The cosmetic composition of 6, further comprising (B) an oil-soluble UV absorber.
8. The cosmetic composition of 7 wherein the amount of component (B) is at least 4% by weight of the composition.
9. The cosmetic composition of any one of 6 to 8, further comprising (C) a powder.
10. The cosmetic composition of 9 wherein the amount of component (C) is at least 2% by weight of the composition.
11. The cosmetic composition of any one of 6 to 10, further comprising (D) an oil having a kinematic viscosity of 1 to 100 mm²/s at 25°C.

### ADVANTAGEOUS EFFECTS OF INVENTION

The organopolysiloxane of the invention exhibits satisfactory dispersibility and dispersion stability when blended in a powder-containing composition, and offers a cosmetic composition having a high age stability and satisfactory spreadability and skin-adaptability when blended in a cosmetic composition, despite of containment of an oil-soluble UV absorber.

### DESCRIPTION OF EMBODIMENTS

Now, I. an organopolysiloxane, II. a method of preparing organopolysiloxane, and III. a cosmetic composition comprising the organopolysiloxane are described in sequence.

I. The organopolysiloxane of the invention is an alkyl/glycerin-co-modified branched organopolysiloxane represented by the compositional formula (1):
[Chem. 5]

(R¹₂SiO_{2/2})ₐ(R¹R²SiO_{2/2})_{b}(R¹R³SiO_{2/2})_{c}(R¹R⁴SiO_{2/2})_{d}(R¹SiO_{3/2})ₑ(SiO_{4/2})_{f}(R¹₃SiO_{1/2})_{2+e+2f} (1)

having R¹: monovalent hydrocarbon group selected from C₁-C₅ alkyl groups, C₆-C₁₅ aryl groups, and C₇-C₁₅ aralkyl groups, R²: C₆-C₂₀ alkyl group, R³: specific glycerin group, and R⁴: branched silicone group on the silicone backbone. The organopolysiloxane is described in detail.

R¹ is independently a monovalent hydrocarbon group selected from C₁-C₅ alkyl groups, C₆-C₁₅ aryl groups, and C₇-C₁₅ aralkyl groups. Examples include alkyl groups such as methyl, ethyl, propyl, butyl and pentyl, cycloalkyl groups such as cyclopentyl, aryl groups such as phenyl and tolyl, aralkyl groups such as benzyl and phenethyl, and fluorinated alkyl groups such as trifluoropropyl and heptadecafluorodecyl. Also included are amino-substituted alkyl groups such as 3-aminopropyl and carboxy-substituted alkyl groups such as 3-carboxypropyl. It is also acceptable that different groups are included in the molecule. R¹ is preferably methyl.

R² is a C₆-C₂₀ alkyl group, which may be straight or branched. Examples include hexyl, heptyl, octyl, nonyl, decyl, dodecyl, tetradecyl and hexadecyl. Inter alia, dodecyl and hexadecyl are preferred in view of compatibility, and dodecyl which is an alkyl group of 12 carbon atoms is more preferred.

R³ is a group having the formula (3) which is bonded to a silicon atom in the polysiloxane backbone via a linking group having the formula (2):

-Q-O- (2)

wherein Q is a C₂-C₂₀ divalent hydrocarbon group, or a glycerin group consisting of 2 to 10 glycerol units selected from the formulae (3) to (9), which is bonded to a silicon atom in the polysiloxane backbone via a linking group having the formula (2).

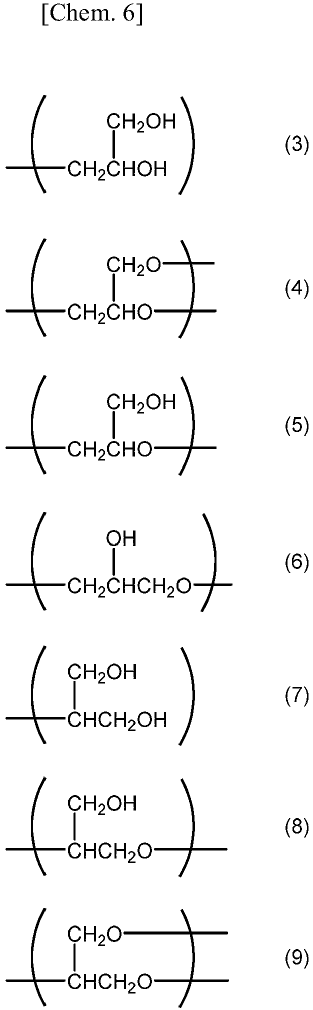

Q is a C₂-C₂₀ divalent hydrocarbon group, examples of which include -(CH₂)₂-, -(CH₂)₃-, -CH₂CH(CH₃)CH₂-, -(CH₂)₄-, -(CH₂)₅-, -(CH₂)₆-, -(CH₂)₇-, -(CH₂)₈-, -(CH₂)₂-CH(CH₂CH₂CH₃)-, and -CH₂-CH(CH₂CH₃)-.

R³ is only one group having formula (3) which is bonded to a silicon atom in the polysiloxane backbone via a linking group having formula (2):

-Q-O- (2),

or a glycerin group consisting of 2 to 10 glycerol units selected from formula (3) and formulae (4) to (9), which is bonded to a silicon atom in the polysiloxane backbone via a linking group having formula (2). The glycerol units may be identical or different. When the glycerin group consists of glycerol units selected from formulae (3) to (9), the number of glycerol units is 2 to 10, preferably 2 to 5, more preferably 2 or 3. If the number of units exceeds 10, the dispersant becomes so polar as to raise problems of degrading the compatibility of the dispersant with an oil, particularly when the oil is a silicone oil. As used herein, the alkyl/glycerin-co-modified branched organopolysiloxane includes monoglycerin and polyglycerins having a plurality of glycerin units condensed as a hydrophilic functional group.

Exemplary groups R³ include groups derived from monoglycerin having the general formula (14), diglycerins having the general formulae (15) and (16), and triglycerins having the general formulae (17) to (20).

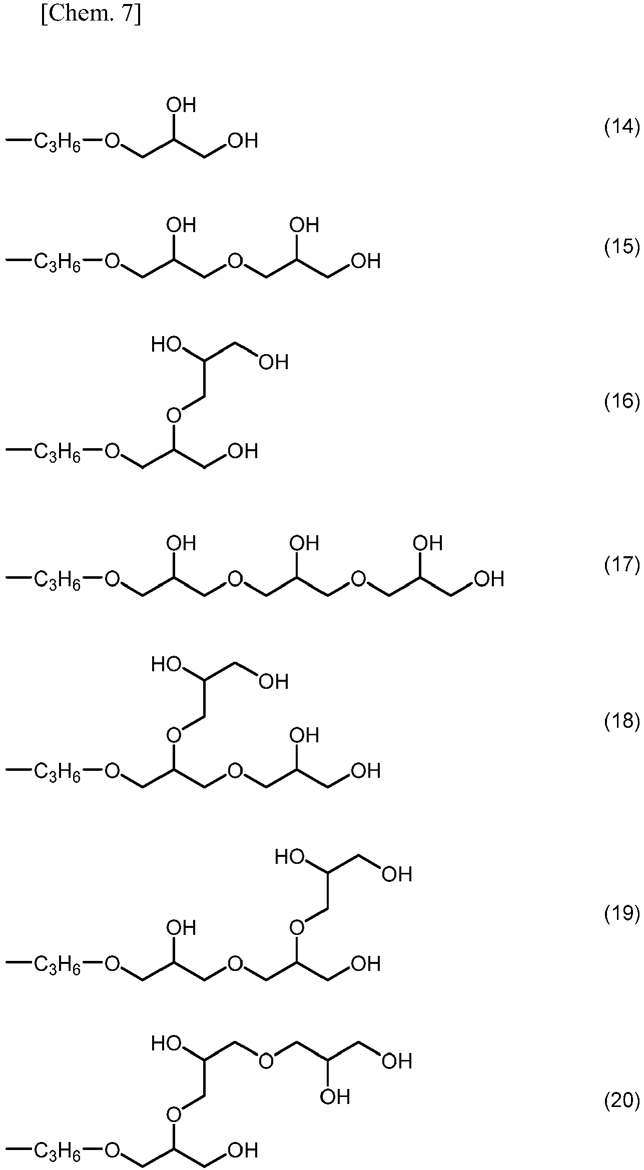

R⁴ is a group having the general formula (10), (11), (12) or (13).
[Chem. 8]

-(CH₂)₂-C_{g}H_{2g}-(SiOR¹₂)ₕ-SiR¹₃ (10)

-(CH₂)₂-C_{g}H_{2g}-SiR¹ₕ₁-(OSiR¹₃)₃₋ₕ₁ (11)

-(CH₂)₂-C_{g}H_{2g}-SiR¹ₕ₁-(OSiR¹ₕ₂(OSiR¹₃)₃₋ₕ₂)₃₋ₕ₁ (12)

-(CH₂)₂-C_{g}H_{2g}-SiR¹ₕ₁-(OSiR¹ₕ₂(OSiR¹ₕ₃(OSiR¹₃)₃₋ₕ₃)₃₋ₕ₂)₃₋ₕ₁ (13)

Herein R¹ is as defined above, g is an integer in the range: 0 ≤ g ≤ 5, and g=0 particularly when the organopolysiloxane is synthesized from reaction of vinylsiloxy group with hydrosilyl group. The subscript h is an integer in the range: 0 ≤ h ≤ 100, preferably from 1 to 30. If h exceeds 100, there arise problems such as degraded adsorption of the dispersant to a powder and degraded dispersion stability with time. The subscripts h1 to h3 each are an integer of 0 to 2.

The subscripts a, b, c, d, e, and f each are a number in the range: 1 ≤ a ≤ 9, 3 < b ≤ 8, 0.4 ≤ c ≤ 3, 0.3 ≤ d ≤ 5, e ≥ 0, and f ≥ 0, meeting 7 ≤ a+b+c+d ≤ 20 and 0.2 ≤ (b+c)/(a+d) ≤ 3. If b is equal to or less than 3 or if b is more than 8, the compatibility of the dispersant with a silicone oil is aggravated and dispersibility is degraded. Preferably b is 4 ≤ b ≤ 8, more preferably 4 ≤ b ≤ 6. If c is more than 3, the dispersant becomes more polar, raising a problem of inviting a degradation of compatibility of the dispersant with an oil, particularly when the oil is a silicone oil. Preferably c is 0.4 ≤ c ≤ 1.4, and 0.4 ≤ d ≤ 1.3.

One of the most important factors in the invention is the value of a+b+c+d. The value of a+b+c+d is 7 ≤ a+b+c+d ≤ 20, preferably 10 ≤ a+b+c+d ≤ 18, more preferably 10 ≤ a+b+c+d ≤ 15. If a+b+c+d is more than 20, the dispersant itself has a high viscosity and is less compatible with an oil and a powder, the amount of substance per weight of the dispersant is reduced, and the surface adsorption to a powder loses uniformity, leading to a degradation of dispersibility. When the organopolysiloxane is used as an emulsifier, the emulsifier itself has a high viscosity and accordingly, the heavy feeling of alkyl group manifests outstandingly. For this reason, the value of a+b+c+d should be up to 20. Moreover, the value of (b+c)/(a+d) is from 0.2 to 3. If the value of (b+c)/(a+d) is less than 0.2, the organopolysiloxane in entirety has a lower proportion of polar groups so that the compatibility with oils such as ester oils and hydrocarbon oils is degraded. On the other hand, if the value of (b+c)/(a+d) is more than 3, not only the compatibility with silicone oils is degraded, but the silicone compound itself has a high viscosity, inviting a degradation of feeling on use or a viscosity buildup of an oil.

The total weight of R² is preferably 20 to 40% by weight, more preferably 20 to 25% by weight of one molecule, and the total weight of R³ is preferably 5 to 20% by weight, more preferably 8 to 15% by weight of one molecule. If the weight of R² or R³ exceeds the upper limit, there is a risk that the compatibility of the dispersant with an oil is aggravated, and dispersibility is degraded. If R² is less than 20% by weight, there is a risk that the compatibility of the dispersant with an organic UV absorber or polar oil is insufficient, failing to disperse the powder in a stable manner.

The organopolysiloxane should have a viscosity of 200 to 3,000 mPa·s, preferably 200 to 1,500 mPa·s as measured at 25°C by a B-type viscometer according to the method of JIS K 7117-1: 1999. If the viscosity exceeds 3,000 mPa·s, the feeling on use becomes worse, the compatibility with oils is degraded, or the dispersion itself experiences a viscosity buildup.

The organopolysiloxane preferably has a weight average molecular weight of 1,500 to 7,500, more preferably 2,000 to 4,000 as viewed from a balance between the viscosity of the dispersant itself and the compatibility with an oil. If the Mw exceeds 7,500, the organopolysiloxane has a higher viscosity, inviting degradation of feeling on use and a viscosity buildup of an oil. In addition, the amount of substance per unit weight is reduced, causing a failure to uniformly treat the powder and a loss of dispersion stability. When the organopolysiloxane is used as an emulsifier, Mw should preferably be up to 7,500 for mitigating the sticky feeling of alkyl and glycerin groups.

It is noted that Mw is as measured by gel permeation chromatography (GPC) versus polystyrene standards under the following conditions.

### [Analysis conditions]

| | |
|---|---|
| Developing solvent: | tetrahydrofuran (THF) |
| Flow rate: | 0.6 mL/min |
| Detector: | differential refractive index detector (RI) |
| Column: | TSK Guardcolumn SuperH-H |
| | TSKgel Super HM-N (6.0 mm I.D. × 15 cm × 1) |
| | TSKgel Super H2500 (6.0 mm I.D. × 15 cm × 1) |
| | (all by Tosoh Corp.) |
| Column temperature: | 40°C |
| Sample injection volume: | 50 µL (THF solution having concentration 0.3% by weight) |

### II. Method of preparing organopolysiloxane

The organopolysiloxane of the invention can be prepared by a standard method, typically by performing addition reaction of an organohydrogenpolysiloxane, an alkylene compound, an allyl ether compound having a glycerin group, and an organopolysiloxane having an unsaturated group such as vinyl, in the presence of a catalyst such as a platinum group metal-based catalyst. Specifically the organopolysiloxane of the invention is obtained by specifying the chain length of the organohydrogenpolysiloxane as one reactant and adjusting the amounts of reactants.

### III. Cosmetic composition comprising organopolysiloxane

The invention also provides a cosmetic composition comprising organopolysiloxane (I) as component (A). As component (A), the organopolysiloxane may be used alone or in admixture of two or more. Hereinafter, the name of ingredients is sometimes expressed by the International Nomenclature for Cosmetic Ingredients (INCI).

The content of component (A) is preferably from 0.1% by weight to less than 15% by weight, more preferably from 0.5% by weight to less than 10% by weight, even more preferably from 1 % by weight to less than 5% by weight of the cosmetic composition. The organopolysiloxane (I) is preferably at least 0.1% by weight in view of a satisfactory surface active effect and less than 15% by weight in view of cosmetic feeling. The following components may be formulated in the cosmetic composition.

### [(B) Oil-soluble UV absorber]

Component (B) is an oil-soluble UV absorber. The oil-soluble UV absorber used herein is not particularly limited as long as it is commonly blended in cosmetics. The absorber may be used alone or in admixture. Examples include homosalate (Japanese labeling name, INCI: Homosalate), octocrylene (labeling name, INCI: Octocrylene), t-butyl methoxydibenzoylmethane (labeling name, INCI: Butyl Methoxydibenzoylmethane), ethylhexyl salicylate (labeling name, INCI: Ethylhexyl Salicylate), diethylaminohydroxybenzoyl hexyl benzoate (labeling name, INCI: Diethylamino Hydroxybenzoyl Hexyl Benzoate), oxybenzone-6 (labeling name, INCI: Benzophenone-6), oxybenzone-9 (labeling name, INCI: Benzophenone-9), oxybenzone-1 (labeling name, INCI: Benzophenone-1), polysilicone-15 (labeling name, INCI: Polysilicone-15), dimethoxybenzylidene dioxoimidazolidine octyl propionate (labeling name, INCI: Ethylhexyl Dimethoxybenzylidene Dioxoimidazolidine Propionate), oxybenzone-2 (labeling name, INCI: Benzophenone-2), terephthalylidene dicamphor sulfonic acid (labeling name, INCI: Terephthalylidene Dicamphor Sulfonic Acid), ethylhexyl triazone (labeling name, INCI: Ethylhexyl Triazone), methylbis(trimethylsiloxy)silyl isopentyl trimethoxycinnamate (labeling name, INCI: Isopentyl Trimethoxycinnamate Trisiloxane), drometrizole trisiloxane (labeling name, INCI: Drometrizole Trisiloxane), dimethyl PABA ethylhexyl (labeling name, INCI: Ethylhexyl Dimethyl PABA), isopropyl para-methoxycinnamate (labeling name, INCI: Isopropyl Methoxycinnamate), ethylhexyl methoxycinnamate (labeling name, INCI: Ethylhexyl Methoxycinnamate), bisethylhexyloxyphenol methoxyphenyltriazine (labeling name, INCI: Bis-Ethylhexyloxyphenol Methoxyphenyl Triazine), oxybenzone-3 (labeling name, INCI: Benzophenone-3), oxybenzone-4 (labeling name, INCI: Benzophenone-4), oxybenzone-5 (labeling name, INCI: Benzophenone-5), phenylbenzimidazole sulfonic acid (labeling name, INCI: Phenylbenzimidazole Sulfonic Acid), methylene bisbenzotriazolyl tetramethylbutylphenol (labeling name, INCI: Methylene Bis-Benzotriazolyl Tetramethylbutylphenol), glyceryl ethylhexanoate dimethoxycinnamate (labeling name, INCI: Glyceryl Ethylhexanoate Dimethoxycinnamate), glyceryl PABA (labeling name, INCI: Glyceryl PABA), methyl diisopropylcinnamate (labeling name, INCI: Diisopropyl Methyl Cinnamate), cinoxate (labeling name, INCI: Cinoxate), ethylhexyl dimethoxybenzylidene dioxoimidazolidine propionate (labeling name, INCI: Ethylhexyl Dimethoxybenzylidene Dioxoimidazolidine Propionate), etc. Also, UVA absorbers (e.g., diethylaminohydroxybenzoyl hexyl benzoate (labeling name, INCI: Diethylamino Hydroxybenzoyl Hexyl Benzoate)) and UVB absorbers (e.g., ethylhexyl methoxcinnamate (labeling name, INCI: Ethylhexyl Methoxycinnamate)) may be used in combination. Also, each of them may be used in admixture.

Preferred particularly in view of compatibility with oils are one or more oil-soluble UV absorbers which are selected from ethylhexyl methoxycinnamate (labeling name, INCI: Ethylhexyl Methoxycinnamate), diethylaminohydroxybenzoyl hexyl benzoate (labeling name, INCI: Diethylamino Hydroxybenzoyl Hexyl Benzoate), octyl salicylate (labeling name, INCI: Ethylhexyl Salicylate), polysilicone-15 (labeling name, INCI: Polysilicone-15), t-butylmethoxydibenzoylmethane (labeling name, INCI: Butyl Methoxydibenzoylmethane), oxybenzone (labeling name, INCI: Benzophenonen-6), methylene bisbenzotriazolyltetramethylbutylphenol (labeling name, INCI: Methylene Bis-Benzotriazolyl Tetramethylbutylphenol), bisethylhexyloxyphenol methoxyphenyl triazine (labeling name, INCI: Bis-Ethylhexyloxyphenol Methoxyphenyl Triazine), homosalate (labeling name, INCI: Homosalate), and octocrylene (labeling name, INCI: Octocrylene).

The content of component (B) is preferably at least 4% by weight, more preferably at least 5% by weight, even more preferably at least 7% by weight of the cosmetic composition. Less than 4% by weight may fail to exert a UV-protecting effect.

### [(C) Powder]

Component (C) is a powder. Suitable powders include microparticulate metal oxide powders, hydrophobized coloring pigments, inorganic powders, metal powders, organic powders, and inorganic/organic composite powders. Specific examples are shown below.

### · Microparticulate metal oxide powder

The microparticulate metal oxide powder used herein is one or more selected from microparticulate titanium oxide (labeling name, INCI: Titanium Dioxide), iron-containing titanium oxide, zinc oxide (labeling name, INCI: Zinc Oxide), cerium oxide (labeling name, INCI: Cerium Oxide), and composites thereof. The metal oxide may also be a powder mixture with another powder. The powder has an average primary particle size of preferably up to 200 nm, more preferably up to 120 nm. If the particle size is larger than the limit, the UV-protecting function is degraded, with white spots being left. It is noted that the average primary particle size can be computed from a photo taken under a transmission electron microscope.

The microparticulate metal oxide powder may be used in untreated state or after a well-known surface treatment commonly used in cosmetics. For example, suitable inorganic treatments include coating with silica (labeling name, INCI: Silica), coating with alumina (labeling name, INCI: Alumina), and coating with Al hydroxide (labeling name, INCI: Aluminum Hydroxide). Suitable organic treatments include coating with silanes or silylating agents such as triethoxycaprylylsilane (labeling name, INCI: Triethoxycaprylylsilane), coating with silicone oils such as dimethicone (labeling name, INCI: Dimethicone), hydrogen dimethicone (labeling name, INCI: Hydrogen Dimethicone), triethoxysilylethyl polydimethylsiloxyethyl hexyl dimethicone (labeling name, INCI: Triethoxysilylethyl Polydimethylsiloxyethyl Hexyl Dimethicone), waxes, paraffins, organic fluorine compounds such as perfluoroalkyl phosphoric acid salts, surfactants, amino acids such as N-acylglutamic acid, and metal soaps such as Al stearate (labeling name, INCI: Aluminum Stearate) and Mg myristate (labeling name, INCI: Magnesium Myristate). In particular, triethoxysilylethyl polydimethylsiloxyethyl hexyl dimethicone (labeling name, INCI: Triethoxysilylethyl Polydimethylsiloxyethyl Hexyl Dimethicone) is applicable to sun-screens and foundations because it exhibits high dispersibility to either of silicones and oils. Treatment with triethoxycaprylylsilane (labeling name, INCI: Triethoxycaprylylsilane) and metal soaps is preferred in view of compatibility with component (B) or component (D) to be described later. These surface treatments may be applied alone or in combination depending on a particular purpose.

As the surface treated microparticulate metal oxide, commercially available products may be used. For example, microparticulate titanium oxide is commercially available under the trade name of STR-100C-LP, STR-100A-LP, STR-100W, STR-100W-LP, STR-100C-LF, STR-40-LP (Sakai Chemical Industry Co., Ltd.), MT-01, MT-05, MT-100Z, MT-100TV, MT-100AQ, MT-150EX, MT-500B, MT-505SAS, MT-700B, MT-014Z, SMT-500SAS (Tayca Corp.), and ST-455, ST-455WS, ST-457ECS and ST-495M (Titan Kogyo Ltd.). Microparticulate zinc oxide is commercially available under the trade name of FINEX-50S-LP2, FINEX-30S-LP2, FINEX-50W, FINEX-30W, FINEX-50W-LP2, FINEX-52W-LP2, FINEX-30W-LP2, FINEX-33W-LP2, FINEX-50-LPT, FINEX-25-LPT, FINEX-50S-LPT, FINEX-30S-LPT (Sakai Chemical Industry Co., Ltd.), MZ-150, MZ-200, MZ-300, MZ-306X, MZ-500HP, MZ-505T, MZ-506X, MZY-203S, MZY-210M3S, TMZ-HA1, MZX-5080TS (Tayca Corp.).

### · Hydrophobized coloring pigments

The coloring pigment for the hydrophobized coloring pigment is not particularly limited as long as it is commonly used in cosmetics for the coloring purpose. Examples include red iron oxide (labeling name, INCI: Iron Oxides), yellow iron oxide (labeling name, INCI: Iron Oxides), white titanium oxide (labeling name, INCI: Titanium Dioxide), black iron oxide (labeling name, INCI: Iron Oxides), Ultramarine (labeling name, INCI: Ultramarines), Prussian blue (labeling name, INCI: Ferric Ferrocyanide, Ferric Ammonium Ferrocyanide), manganese violet (labeling name, INCI: Manganese Violet), cobalt titanate (labeling name, INCI: Cobalt Titanium Oxide), chromium hydroxide (labeling name, INCI: Chromium Hydroxide Green), chromium oxide (labeling name, INCI: Chromium Oxide Greens), Al/cobalt oxide (labeling name, INCI: Cobalt Aluminum Oxide), cobalt titanate (labeling name, INCI: Cobalt Titanium Oxide), titanium/titanium oxide (labeling name, INCI: Titanium/Titanium Dioxide), Li/cobalt titanate (labeling name, INCI: Lithium Cobalt Titanate), cobalt titanate (labeling name, INCI: Cobalt Titanium Oxide), sintered iron oxide/titanium oxide (labeling name, INCI: Iron Oxide/Titanium Dioxide Sinter), composites doped with a different metal such as iron oxide-doped titanium oxide (labeling name, INCI: Iron Oxides, Titanium Dioxide), titanium nitride (labeling name, INCI: Titanium Nitride), ferrous hydroxide (labeling name, INCI: Iron Hydroxide), inorganic brown pigments such as γ-iron oxide, inorganic yellow pigments such as loess, colored pigments such as lake form of tar dyes and lake form of natural dyes. Any of the foregoing may be used.

The hydrophobized coloring pigment may take any shape including spherical, generally spherical, bar, spindle, petal, strip, and irregular shapes. Its geometry is not particularly limited as long as a color can be imparted to a cosmetic composition. In view of hiding power, a pigment having a particle size, specifically a volume average particle size in the range of 150 to 600 nm is preferred. The volume average particle size may be measured by means of TEM or the like. If the particle size is less than 150 nm, the hiding power is so low that the cosmetic composition may have a low coloring efficiency. If the particle size is more than 600 nm, the feeling on use may be aggravated.

Moreover, the pigment used herein may be treated on parts or the entirety of its surface with inorganic compounds such as alumina (labeling name, INCI: Alumina), aluminum hydroxide (labeling name, INCI: Aluminum Hydroxide), silica (labeling name, INCI: Silica), and hydrous silica (labeling name, INCI: Hydrated silica).

The hydrophobizing treatment for the hydrophobized coloring pigment refers to surface treatment of the coloring pigment with a hydrophobizing agent. The hydrophobizing agent for the coloring pigment is not particularly limited as long as it can impart hydrophobicity. Exemplary treating agents include silicone treating agents, waxes, paraffins, organic fluorine compounds such as perfluoroalkyl phosphate salts, surfactants, amino acids such as N-acylglutamic acid, and metal soaps such as Al stearate (labeling name, INCI: Aluminum Stearate) and Mg myristate (labeling name, INCI: Magnesium Myristate).

Among others, the silicone treating agents are preferably used. Included are silanes or silylating agents such as triethoxycaprylylsilane (labeling name, INCI: Triethoxycaprylylsilane) or trimethoxysilyl dimethicone (labeling name, INCI: Trimethoxysilyl Dimethicone); silicone oils such as dimethicone (labeling name, INCI: Dimethicone), hydrogendimethicone (labeling name, INCI: Hydrogen Dimethicone), triethoxysilylethyl polydimethylsiloxyethyl hexyl dimethicone (labeling name, INCI: Triethoxysilylethyl Polydimethylsiloxyethyl Hexyl Dimethicone); and silicone compounds such as acrylate/tridecyl acrylate/triethoxysilylpropyl methacrylate/dimethicone methacrylate copolymers (labeling name, INCI: Acrylate/Tridecyl Acrylate/Triethoxysilylpropyl Methacrylate/Dimethicone Methacrylate Copolymer), acrylate/dimethicone copolymer (labeling name, INCI: Acrylate/Dimethicone Copolymer). As the silicone treating agent, the silicone powder treating agents described in JP 3912961 are preferably used. In particular, triethoxysilylethyl polydimethylsiloxyethyl hexyl dimethicone (labeling name, INCI: Triethoxysilylethyl Polydimethylsiloxyethyl Hexyl Dimethicone) which is a dimethylpolysiloxane having triethoxysilyl, polydimethylsiloxyethyl and hexyl groups on side chains, or the like is advantageously used because it exerts a high affinity even when the dispersing medium in which the highly hydrophobized coloring pigment is dispersed is a mixture of silicone and hydrocarbon. It is noted that the surface hydrophobizing agent may be used alone or in admixture of two or more.

In the practice of the invention, the method of surface treating the coloring pigment using the hydrophobizing agent is not particularly limited and may be performed by any well-known methods. The surface treating method is generally classified into a dry mode and a wet mode. In the dry mode, treatment can be performed, for example, by using a Henschel mixer, ball mill, jet mill, kneader, planetary mixer, sand mill, attritor, ribbon blender, disper mixer, homomixer, arbitrary agitators, crusher, mixer, disperser or the like, and mixing and contacting the coloring pigment with the hydrophobizing agent. At this point, treatment may be performed while imparting energy such as heat, mechanochemical mechanical forces or overheated steam. It is also acceptable that after the coloring pigment and the hydrophobizing agent are fully mixed and contacted, treatment is performed by imparting energy such as heat, mechanochemical mechanical forces or overheated steam. Alternatively, in the step of mixing and contacting the coloring pigment with the hydrophobizing agent, the hydrophobizing agent may be previously dissolved or dispersed in an arbitrary amount of water, solvent or supercritical fluid, and the resulting solution or liquid be sprayed to the coloring pigment, for the purpose of increasing the dispersion efficiency of the hydrophobizing agent. The treatment in wet mode may be performed by dispersing the coloring pigment and the hydrophobizing agent in water, solvent or supercritical fluid, for thereby mixing and contacting the coloring pigment with the hydrophobizing agent, and thereafter, evaporating off the solvent, and further separately imparting energy such as heat, mechanochemical mechanical forces or overheated steam.

Examples of the coloring pigment having undergone hydrophobic surface treatment include KTP-09 series, especially KTP-09W, KTP-09R, KTP-09Y and KTP-09B (Shin-Etsu Chemical Co., Ltd.).

### · Inorganic powder

Examples of the inorganic powder include microparticles of zirconium oxide (labeling name, INCI: Zirconium Dioxide), zinc oxide (labeling name, INCI: Zinc Oxide), cerium oxide (labeling name, INCI: Cerium Oxide), magnesium oxide (labeling name, INCI: Magnesium Oxide), barium sulfate (labeling name, INCI: Barium Sulfate), calcium sulfate (labeling name, INCI: Calcium Sulfate), magnesium sulfate (labeling name, INCI: Magnesium Sulfate), calcium carbonate (labeling name, INCI: Calcium Carbonate), magnesium carbonate (labeling name, INCI: Magnesium Carbonate), talc (labeling name, INCI: Talc), cleaved talc (labeling name, INCI: Talc), mica (labeling name, INCI: Mica), kaolin (labeling name, INCI: Kaolin), cericite (labeling name, INCI: Mica), synthetic fluorphlogopite (labeling name, INCI: Synthetic Fluorphlogopite), black mica (labeling name, INCI: Biotite), potassium silicate (labeling name, INCI: Potassium Silicate), silica (labeling name, INCI: Silica), fumed silica, aluminum silicate (labeling name, INCI: Aluminum Silicate), magnesium silicate (labeling name, INCI: Magnesium Silicate), Al/Mg silicate (labeling name, INCI: Magnesium Aluminum Silicate), calcium silicate (labeling name, INCI: Calcium Silicate), Al/Ca/Na silicate (labeling name, INCI: Aluminum Calcium Sodium silicate), Li/Mg/Na silicate (labeling name, INCI: Lithium Magnesium Sodium Silicate), Na/Mg silicate (labeling name, INCI: Sodium Magnesium Silicate), Ca/Al borosilicate (labeling name, INCI: Calcium Aluminum Borosilicate), Ca/Na borosilicate (labeling name, INCI: Calcium Sodium Borosilicate), hydroxyapatite (labeling name, INCI: Hydroxyapatite), bentonite (labeling name, INCI: Bentonite), montmorillonite (labeling name, INCI: Montmorillonite), hectorite (labeling name, INCI: Hectorite), zeolite (labeling name, INCI: Zeolite), alumina (labeling name, INCI: Alumina), Al hydroxide (labeling name, INCI: Aluminum Hydroxide), boron nitride (labeling name, INCI: Boron Nitride), and glass (labeling name, INCI: Glass). Also, examples of the inorganic coloring pearly pigment include pearlescent pigments such as titanium oxide-coated mica, and bismuth oxychloride (labeling name, INCI: Bismuth Oxychloride), bismuth oxychloride (labeling name, INCI: Bismuth Oxychloride) coated with titanium oxide (labeling name, INCI: Titanium Dioxide), talc (labeling name, INCI: Talc) coated with titanium oxide (labeling name, INCI: Titanium Dioxide), fish scale flakes, coloring mica coated with titanium oxide (labeling name, INCI: Titanium Dioxide), which may either be untreated or have undergone well-known surface treatment commonly used for cosmetics.

### · Metal powder

Examples of the metal powder include metal microparticles such as aluminum (labeling name, INCI: Aluminum Powder), copper (labeling name, INCI: Copper Powder), and silver (labeling name, INCI: Silver Powder).

### · Organic powder

Examples of the organic powder include silicone, polyamide, polyacrylic acid-acrylate, polyester, polyethylene, polypropylene, polystyrene, styrene-acrylic acid copolymers, divinylbenzene-styrene copolymers, polyurethane, vinyl resins, urea resins, melamine resins, benzoguanamine, polymethyl benzoguanamine, tetrafluoroethylene, polymethyl methacrylate (e.g., polymethyl methacrylate), cellulose, silk, nylon, phenolic resins, epoxy resins, polycarbonate, etc. in powder form. In particular, as the silicone, silicone resin particles, e.g., polymethylsilsesquioxane (labeling name, INCI: Polymethylsilsesquioxane), and silicone resin-coated silicone rubber powder (e.g., (vinyldimethicone/methicone silsesquioxane) crosspolymer (labeling name, INCI: Vinyl Dimethicone/Methicone Silsesquioxane Crosspolymer), diphenyl dimethicone/vinyldiphenyl dimethicone/silsesquioxane) crosspolymer (labeling name, INCI: Diphenyl Dimethicone/Vinyl Diphenyl Dimethicone/Silsesquioxane Crosspolymer), polysilicone-1 crosspolymer (labeling name, INCI: Polysilicone-1 Crosspolymer), polysilicone-22 (labeling name, INCI: Polysilicone-22). Metal soaps are also included, examples of which include zinc stearate (labeling name, INCI: Zinc Stearate), Al stearate (labeling name, INCI: Aluminum Stearate), Ca stearate (labeling name, INCI: Calcium Stearate), Mg stearate (labeling name, INCI: Magnesium Stearate), zinc myristate (labeling name, INCI: Zinc Myristate), Mg myristate (labeling name, INCI: Magnesium Myristate), Zn/Na cetylphosphate (labeling name, INCI: Sodium Zinc Cetyl Phosphate), K cetylphosphate (labeling name, INCI: Potassium Cetyl Phosphate) in powder form. Also included are organic colorants, examples of which include tar dyes, specifically Red #3, Red #104(1) (labeling name, INCI: Red 28, Red 28 Lake), Red #201 (labeling name, INCI: Red 6), Red #202 (labeling name, INCI: Red 7), Red #204, Red #205, Red #220 (labeling name, INCI: Red 34), Red #226 (labeling name, INCI: Red 30), Red #227 (labeling name, INCI: Red 33, Red 33 Lake), Red #228 (labeling name, INCI: Red 36), Red #230(1) (labeling name, INCI: Red 22, Red 22 Lake), Red #230(2), Red #401, Red #505, Yellow #4 (labeling name, INCI: Yellow 5), Yellow #5 (labeling name, INCI: Yellow 6, Yellow 6 Lake), Yellow #202(1) (labeling name, INCI: Yellow 8), Yellow #203 (labeling name, INCI: Yellow 10, Yellow 10 Lake), Yellow #204 (labeling name, INCI: Yellow 11), Yellow #401 (labeling name), Blue #1 (labeling name, INCI: Blue 1 Lake), Blue #2, Blue #201, Blue #205 (labeling name, INCI: Blue 4), Blue #404 (labeling name, INCI: Blue 4), Blue #404, Green #3 (labeling name, INCI: Green 3, Green 3 Lake), Green #201 (labeling name, INCI: Green 5), Green #202 (labeling name, INCI: Green 6), Green #204 (labeling name, INCI: Green 8), Green #205, Orange #201 ((labeling name, INCI: Orange 5), Orange #203 (labeling name, INCI: Pigment Orange 5), Orange #204, Orange #205 (labeling name, INCI: Orange 4, Orange 4 Lake), Orange #206 (labeling name, INCI: Orange 10), Orange #207 (labeling name, INCI: Orange 11); and natural dyes, specifically cochineal (labeling name, INCI: Cochineal), laccaic acid (labeling name, INCI: Laccaic Acid), safflower red (labeling name, INCI: Garthamus Tinctorius (Safflower) Flower Extract), red root extract (labeling name, INCI: Lithospermum Officinale Root Extract), gardenia yellow, and gardenia blue (labeling name, INCI: Hydrolyzed Gardenia Florida Extract).

### · Inorganic/organic composite powder

Exemplary of the inorganic/organic composite powder is a composite powder obtained by coating an inorganic powder on the surface with an organic powder by any well-known method.

The content of component (C) is preferably at least 2% by weight, more preferably at least 3% by weight, even more preferably at least 10% by weight of the cosmetic composition. Less than 2% by weight may fail to achieve a satisfactory hiding effect or coloring effect.

### [(D) Oil]

Component (D) is an oil having a kinematic viscosity of 1 to 100 mm²/s at 25°C, which may be used alone or in admixture. As used herein, the kinematic viscosity is measured at 25°C by a Cannon-Fenske viscometer according to JIS Z 8803: 2011. An oil having a kinematic viscosity of 1 to 20 mm²/s is more preferred. An oil having a kinematic viscosity of up to 100 mm²/s is preferred in view of compatibility with component (A). Examples of the oil as component (D) include hydrocarbon oils, higher fatty acids, higher alcohols, esters, silicone oils and fluorochemical oils. They are specifically described below.

### · Hydrocarbon oil

The hydrocarbon oils include straight or branched hydrocarbon oils while they may be either volatile or nonvolatile. Examples of the hydrocarbon oil include olefin oligomers, isododecane (labeling name, INCI: Isododecane), dodecane (labeling name, INCI: Dodecane), isohexadecane (labeling name, INCI: Isohexadecane), undecane (labeling name, INCI: Undecane), squalane (labeling name, INCI: Squalane), squalene (labeling name, INCI: Squalene), mineral oil (labeling name, INCI: Mineral Oil), fluid isoparaffin, polyisobutylene (labeling name), hydrogenated polyisobutene (labeling name, INCI: Hydrogenated Polyisobutene), and (C13-15) alkane (labeling name, INCI: C13-15 Alkane). Of these, dodecane (labeling name, INCI: Dodecane) and (C13-15) alkane (labeling name, INCI: C13-15 Alkane) are preferred in view of compatibility with components (A) and (B).

### · Higher fatty acid

Examples of the higher fatty acid include oleic acid (labeling name, INCI: Oleic Acid), linoleic acid (labeling name, INCI: Linoleic Acid), linolenic acid (labeling name, INCI: Linolenic Acid), arachidonic acid (labeling name, INCI: Arachidonic Acid), eicosapentaenoic acid (EPA) (labeling name, INCI: Eicosapentaenoic Acid), docosahexaenoic acid (labeling name, INCI: Docosahexaenoic Acid), isostearic acid (labeling name, INCI: Isostearic Acid), and hydroxystearic acid (labeling name, INCI: Hydroxystearic Acid).

### · Natural animal and plant oils and fats and semi-synthetic oils and fats

Examples of the natural animal and plant oils and fats and semi-synthetic oils and fats include avocado oil (labeling name, INCI: Persea Gratissima (Avocado) Oil), linseed oil (labeling name, INCI: Linum Usitatissimum (Linseed) Seed Oil), almond oil (labeling name, INCI: Prunus Amygdalus Dulcis (Sweet Almond) Oil), olive oil (labeling name, INCI: Olea Europaea (Olive) Fruit Oil), Torreya California oil (labeling name, INCI: Torreya Californica (Calfornia Nutmeg) Oil), citronella grass oil (labeling name, INCI: Cymbopogon Nardus (Citronella) Oil), shark liver oil (labeling name, INCI: Shark Liver Oil), cod liver oil (labeling name, INCI: Cod Liver Oil), fish liver oil (labeling name, INCI: Fish Liver Oil), kyounin oil (labeling name, INCI: Kyounin Yu), sesame oil (labeling name, INCI: Sesamum Indicum (Sesame) Seed Oil), rice germ oil (labeling name, INCI: Oryza Sative (Rice) Germ Oil), rice bran oil (labeling name, INCI: Oryza Sativa (Rice) Bran Oil), camellia oil (labeling name, INCI: Camellia Kissi Seed Oil), safflower oil (labeling name, INCI: Carthamus Tinctorius (Safflower) Seed Oil), turtle oil (labeling name, INCI: Turtle Oil), Japanese camellia oil (labeling name, INCI: Camellia Japonica Seed Oil), primrose oil (labeling name, INCI: Oenothera Biennis (Evening Primrose) Oil), corn germ oil (labeling name, INCI: Zea Mays (Corn) Germ Oil), rapeseed oil (labeling name, INCI: Rape Shushi Yu), wheat germ oil (labeling name, INCI: Triticum Vulgare (Wheat) Germ Oil), persic oil (labeling name), palm oil (labeling name, INCI: Elaeis Guineensis (Palm) Oil), palm kernel oil (labeling name, INCI: Elaeis Guineensis (Palm) Kernel Oil), castor oil (labeling name, INCI: Ricinus Communis (Castor) Seed Oil), hydrogenated castor oil (labeling name), sunflower oil (labeling name, INCI: Helianthus Annuus (Sunflower) Seed Oil), grape seed oil (labeling name, INCI: Vitis Vinifera (Grape) Seed Oil), jojoba seed oil (labeling name, INCI: Simmondsia Chinensis (Jojoba) Seed Oil), macadamia nut oil (labeling name, INCI: Macadamia Ternifolia Seed Oil), mink oil (labeling name, INCI: Mink Oil), meadowfoam oil (labeling name, INCI: Limnanthes Alba (Meadowfoam) Seed Oil), cotton seed oil (labeling name, INCI: Gossypium Herbaceum (Cotton) Seed Oil), coconut oil (labeling name, INCI: Cocos Nucifera (Coconut) Oil), hydrogenated coconut oil (labeling name, INCI: Hydrogenated Coconut Oil), and egg oil (labeling name, INCI: Egg Oil).

### · Ester

The ester oils are liquid oils in condensed forms of fatty acids of 1 to 20 carbon atoms with alcohols of 1 to 20 carbon atoms and may be monoesters, or polyesters such as diesters and triesters. Examples include diisobutyl adipate (labeling name, INCI: Diisobutyl Adipate), dihexyldecyl adipate (labeling name), diheptylundecyl adipate (labeling name, INCI: Diheptylundecyl Adipate), n-alkyl glycol monoisostearates such as isostearyl isostearate (labeling name, INCI: Isostearyl Isostearate), isocetyl isostearate (labeling name, INCI: Isocetyl Isostearate), trimethylolpropane triisostearate (labeling name, INCI: Trimethylolpropane Triisostearate), glycol diethylhexanoate (labeling name, INCI: Glycol Diethylhexanoate), cetyl ethylhexanoate (labeling name, INCI: Cetyl Ethylhexanoate), triethylhexanoin (labeling name, INCI: Triethylhexanoin), trimethylolpropane triethylhexanoate (labeling name, INCI: Trimethylolpropane Triethylhexanoate), pentaerythrityl tetraethylhexanoate (labeling name, INCI: Pentaerythrityl Tetraethylhexanoate), cetyl octanoate (labeling name, INCI: Cetyl Ethylhexanoate), octyldodecyl esters such as octyldodecyl stearoyloxystearate (labeling name, INCI: Octyldodecyl Stearoyl Stearate), oleyl oleate (labeling name, INCI: Oleyl Oleate), octyldodecyl oleate (labeling name, INCI: Octyldodecyl Oleate), decyl oleate (labeling name, INCI: Decyl Oleate), neopentyl glycol dioctanoate (labeling name, INCI: Neopentyl Glycol Diethylhexanoate), neopentyl glycol dicaprate (labeling name, INCI: Neopentyl Glycol Dicaprate), triethyl citrate (labeling name, INCI: Triethyl Citrate), diethylhexyl succinate (labeling name, INCI: Diethylhexyl Succinate), amyl acetate (labeling name, INCI: Amyl Acetate), ethyl acetate (labeling name, INCI: Ethyl Acetate), butyl acetate (labeling name, INCI: Butyl Acetate), isocetyl stearate (labeling name, INCI: Isocetyl Stearate), butyl stearate (labeling name, INCI: Butyl Stearate), diisopropyl sebacate (labeling name, INCI: Diisopropyl Sebacate), diethylhexyl sebacate (labeling name, INCI: Diethylhexyl Sebacate), cetyl lactate (labeling name, INCI: Cetyl Lactate), myristyl lactate (labeling name, INCI: Myristyl Lactate), isononyl isononanoate (labeling name, INCI: Isononyl Isononanoate), isotridecyl isononanoate (labeling name, INCI: Isotridecyl Isononanoate), palmitates such as isopropyl palmitate (labeling name, INCI: Isopropyl Palmitate), ethylhexyl palmitate (labeling name, INCI: Ethylhexyl Isopalmitate), and hexyldecyl palmitate (labeling name, INCI: Isocetyl Palmitate, Hexyldecyl Palmitate), cholesteryl hydroxystearate (labeling name, INCI: Cholesteryl Hydroxystearate), myristates such as isopropyl myristate (labeling name, INCI: Isopropyl Myristate), octyldodecyl myristate (labeling name, INCI: Octyldodecyl Myristate), and myristyl myristate (labeling name, INCI: Myristyl Myristate), ethylhexyl laurate (labeling name, INCI: Ethylhexyl Laurate), hexyl laurate (labeling name, INCI: Hexyl Laurate), dioctyldodecyl lauroyl glutamate (labeling name, INCI: Dioctyldodecyl Lauroyl Glutamate), isopropyl lauroyl sarcosinate (labeling name, INCI: Isopropyl Lauroyl Sarcosinate), diisostearyl malate (labeling name, INCI: Diisostearyl Malate), and glyceride oils such as glyceryl acetate (labeling name, INCI: Glyceryl Acetate) and glyceryl stearate (labeling name, INCI: Glyceryl Stearate). Among these, cetyl ethylhexanoate (labeling name, INCI: Cetyl Ethylhexanoate), glyceryl tri-2-ethylhexanoate (labeling name), neopentyl glycol dioctanoate (labeling name, INCI: Neopentyl Glycol Diethylhexanoate), isononyl isononanoate (labeling name, INCI: Isononyl Isononanoate), isotridecyl isononanoate (labeling name, INCI: Isotridecyl Isononanoate), and ethylhexyl palmitate (labeling name, INCI: Ethylhexyl Palmitate) are preferred in view of compatibility with components (A) and (B).

### · Silicone oil

Examples of the silicone oil include straight or branched dimethicones having a low to high viscosity such as trisiloxane (labeling name, INCI: Trisiloxane), volatile dimethicone (labeling name, INCI: Dimethicone), low viscosity dimethicone (labeling name, INCI: Dimethicone), cyclotetrasiloxane (labeling name, INCI: Cyclotetrasiloxane), cyclopentasiloxane (labeling name, INCI: Cyclopentasiloxane), cyclohexasiloxane (labeling name, INCI: Cyclohexasiloxane), methyl trimethicone (labeling name, INCI: Methyl Trimethicone), caprylyl methicone (labeling name, INCI: Caprylyl Methicone), phenyl trimethicone (labeling name, INCI: Phenyl Trimethicone), methylphenylpolysiloxane (labeling name, INCI: Diphenyl Dimethicone), diphenylsiloxyphenyl trimethicone (labeling name, INCI: Diphenylsiloxy Phenyl Trimethicone), ethyl methicone (labeling name, INCI: Ethyl Methicone), ethyltrisiloxane (labeling name, INCI: Ethyl Trisiloxane), and hydrogen dimethicone (labeling name, INCI: Hydrogen Dimethicone); amodimethicone (labeling name, INCI: Amodimethicone), aminopropyl dimethicone (labeling name, INCI: Aminopropyl Dimethicone), high degree-of-polymerization gum-like dimethicone (labeling name, INCI: Dimethicone), gum-like amodimethicone (labeling name, INCI: Amodimethicone), silicone rubbers such as gum-like dimethylsiloxane-methylphenylsiloxane copolymers, cyclic organopolysiloxane solutions of silicone gums and rubbers, amino acid-modified silicones, fluorine-modified silicones, silicone resins, silicone resin solutions. Of these, methyl trimethicone (labeling name, INCI: Methyl Trimethicone), trisiloxane (labeling name, INCI: Trisiloxane), volatile dimethicone (labeling name, INCI: Dimethicone), low viscosity dimethicone (labeling name, INCI: Dimethicone), and diphenylsiloxyphenyl trimethicone (labeling name, INCI: Diphenylsiloxy Phenyl Trimethicone) are preferred in view of compatibility with components (A) and (B).

### · Fluorochemical oils

Examples of the fluorochemical oil include perfluropolyethers such as polyperfluoromethyl isopropyl ether (labeling name, INCI: Polyperfluoromkethylisopropyl Ether), and perflurocarbons such as perfluorodecalin (labeling name, INCI: Perfluorodecalin) and perfluorohexane (labeling name, INCI: Perfluorohexane).

When component (D) is contained, it may be blended in the cosmetic composition like other components without any pre-treatment. When higher dispersibility in the cosmetic composition is desired, it is preferred to prepare a dispersion by premixing component (D) with other components, and blend the dispersion in the cosmetic composition. As the method of preparing the dispersion, a dispersion is preferably obtained by premixing component (C) with components (A) and (B), components (A) and (D), or components (A), (B) and (D). The step of premixing component (C) with components (A) and (D) is especially preferred in view of dispersibility. Moreover, the dispersion can be prepared by the dispersing step to be described later.

When component (C) is blended, it may be blended in the cosmetic composition like other components without any pre-treatment. When higher dispersibility in the cosmetic composition is desired, it is preferred to prepare a dispersion by premixing component (C) with other components, and blend the dispersion in the cosmetic composition. As the method of preparing the dispersion, a dispersion is preferably obtained by premixing component (C) with components (A) and (B), components (A) and (D), or components (A), (B) and (D). The step of premixing component (C) with components (A) and (D) is especially preferred in view of dispersibility. Moreover, the dispersion can be prepared by the dispersing step to be described later.

The content of component (D) is preferably 5 to 95% by weight, more preferably 10 to 90% by weight of the cosmetic composition.

In the cosmetic composition of the invention, optional components other than the aforementioned components may be blended in appropriate amounts as long as the benefits of the invention are not impaired. Other optional components include, for example, (1) an oily component other than components (B) and (D), (2) a compound having an alcoholic hydroxyl group, (3) a surfactant other than component (A), (4) a mixture consisting of a crosslinked organopolysiloxane and an oil which is liquid at 25°C, (5) a film-forming agent, (6) an antiperspirant, (7) an antibacterial agent, and (8) another additive. These components may be used alone or in admixture.

### (1) Oily component other than components (B) and (D)

The oily component other than components (B) and (D) may be either solid or semi-solid. For example, higher fatty acids, higher alcohols, esters, silicone oils, and fluorochemical oils may be used.

### · Solid oily component

When it is desired that the cosmetic composition be solidified, waxes, hydrocarbons, esters, higher alcohols, and higher fatty acids which are solid at 25°C are preferably blended. Examples of the oily component which is solid at 25°C include waxes, hydrocarbons, esters, higher alcohols, and higher fatty acids having a melting point of preferably at least 40°C, more preferably 60 to 110°C. The oily component is not particularly limited as long as it is commonly blended in cosmetics. Specific examples include plant waxes such as carnauba wax, candelilla wax, rice wax, Japan wax, and shea butter; animal waxes such as beeswax and sperm wax; hydrocarbon waxes such as solid paraffin, polyethylene, ceresin, ozokerite, and microcrystalline wax; higher alcohols such as stearyl alcohol, behenyl alcohol and cetanol; fatty acids such as stearic acid and behenic acid; and silicone waxes such as acrylate silicone resins in the form of acrylate silicone graft or block copolymers (acrylate silicone graft copolymers: KP-561P, 562P by Shin-Etsu Chemical Co., Ltd.) or derivatives thereof.

### · Semisolid oily component

When it is desired to provide the cosmetic composition with a feeling of film thickness, hydrocarbons and esters which are pasty at 25°C are preferably blended. The semisolid oily component is not particularly limited as long as it is commonly blended in cosmetics. Specific examples include lanolin, vaseline, dipentaerythritol fatty acid esters, and hydrogenated oils.

### (2) Compound having alcoholic hydroxyl group

Examples of the compound having an alcoholic hydroxyl group include lower alcohols of 2 to 5 carbon atoms such as ethanol and isopropanol, and sucrose alcohols such as sorbitol and maltose. Also included are sterols such as choresterol, sitosterol, phytosterol, and lanosterol.

### (3) Surfactant other than component (A)

The surfactant other than component (A) may be nonionic, anionic, cationic or ampholytic. The surfactant is not particularly limited as long as it is commonly blended in cosmetics. Of the surfactants, preferred are partially crosslinked polyether-modified silicones, partially crosslinked polyglycerin-modified silicones, straight or branched polyoxyethylene-modified organopolysiloxanes, straight or branched polyoxyethylenepolyoxypropylene-modified organopolysiloxanes, straight or branched polyoxyethylene/alkyl-co-modified organopolysiloxanes, straight or branched polyoxyethylenepolyoxypropylene/alkyl-co-modified organopolysiloxanes, straight or branched polyglycerin-modified organopolysiloxanes, straight or branched polyglycerin/alkyl-co-modified organopolysiloxanes, and pyrrolidone-modified organopolysiloxanes. In these surfactants, the content of hydrophilic polyoxyethylene groups, polyoxyethylenepolyoxypropylene groups or polyglycerin residues is preferably 10 to 70% by weight of the molecule. On use of partially crosslinked polyether-modified silicones or partially crosslinked polyglycerin-modified silicones, it is preferred that in a composition consisting of the crosslinked organopolysiloxane and an oil which is liquid at 25°C, the crosslinked organopolysiloxane swells with the liquid oil as a result of being impregnated with an amount of more than its own weight of the liquid oil. Suitable liquid oils used herein include liquid silicones, hydrocarbon oils, ester oils, natural animal and plant oils, semi-synthetic oils and fluorochemical oils, all included in the optional oil. Illustrative examples include low viscosity silicone having a kinematic viscosity of 0.65 to 100 mm²/s at 25°C, liquid paraffin, squalane, hydrocarbon oils such as isododecane and isohexadecane, glyceride oils such as trioctanoin, ester oils such as isotridecyl isononanoate, N-acylglutamates, and lauroylsarcosinates, and natural animal and plant oils such as macadamia nut oil. Specific examples include KSG-210, KSG-240, KSG-310, KSG-320, KSG-330, KSG-340, KSG-320Z, KSG-350Z, KSG-710, KSG-810, KSG-820, KSG-830, KSG-840, KSG-820Z, and KSG-850Z, all from Shin-Etsu Chemical Co., Ltd. Examples of the surfactant other than the crosslinked organopolysiloxane include KF-6011, KF-6013, KF-6043, KF-6017, KF-6028, KF-6038, KF-6048, KF-6100, KF-6104, KF-6105, and KF-6106, all from Shin-Etsu Chemical Co., Ltd. For any of the foregoing surfactants, the content thereof is preferably 0.1 to 20% by weight of the cosmetic composition. A surfactant content of at least 0.1% by weight enables to fully exert the function of dispersion or emulsification whereas a surfactant content of up to 20% by weight eliminates the risk that the cosmetic composition has a sticky feeling on use. For the purpose of maintaining the cosmetic composition water resistant, the surfactant preferably has a HLB value of 2 to 14.5 although the HLB is not particularly limited.

### (4) Mixture consisting of a crosslinked organopolysiloxane and an oil which is liquid at 25°C

In the mixture consisting of a crosslinked organopolysiloxane and an oil which is liquid at 25°C, preferably the crosslinked organopolysiloxane swells with the liquid oil as a result of being impregnated with an amount of more than its own weight of the liquid oil. Suitable liquid oils used herein include liquid silicones, hydrocarbon oils, ester oils, natural animal and plant oils, semi-synthetic oils, and fluorochemical oils, all included in the optional oil. Illustrative examples include low viscosity silicone having a kinematic viscosity of 0.65 to 100 mm²/s at 25°C, liquid paraffin, squalane, hydrocarbon oils such as isododecane and isohexadecane, glyceride oils such as trioctanoin, ester oils such as isotridecyl isononanoate, N-acylglutamates, and lauroylsarcosinates, and natural animal and plant oils such as macadamia nut oil. Unlike component (3), component (4) is a compound having neither polyether nor polyglycerin structure in the molecule. Specific examples include KSG series available from Shin-Etsu Chemical Co., Ltd., e.g., KSG-15, KSG-16, KSG-016F, KSG-19, KSG-41, KSG-42, KSG-43, KSG-44, KSG-042Z, and KSG-045Z.

### (5) Film-forming agent

The film-forming agent is blended for the purpose of further maintaining the effect-sustainability of the cosmetic composition. A silicone based agent is preferred from the aspect of imparting water repellency though the agent is not limited thereto. Examples of the film-forming agent used herein include trimethylsiloxysilicic acid (labeling name, INCI: trimethylsiloxysilicate), acrylate silicone film-forming agent, silicone-modified norbornene, and silicone-modified pullulan. The film-forming agent may be previously dissolved in an oil which is liquid at room temperature before it is blended in the cosmetic composition. Suitable liquid oils used herein include liquid silicones, hydrocarbon oils, ester oils, natural animal and plant oils, semi-synthetic oils, and fluorochemical oils, all included in the optional oil. Illustrative examples include low viscosity silicone having a kinematic viscosity of 0.65 to 100 mm2/s at 25°C, liquid paraffin, squalane, hydrocarbon oils such as isododecane and isohexadecane, glyceride oils such as trioctanoin, ester oils such as isotridecyl isononanoate, N-acylglutamates, and lauroylsarcosinates, and natural animal and plant oils such as macadamia nut oil. Specific examples include KF-7312J which is a silicone solution of trimethylsiloxysilicic acid, KP-545 and KP-549 which are silicone solutions of acrylate silicone film-former, NBN-30-ID which is an isododecane solution of silicone-modified norbornene, TSPL-30-ID which is an isododecane solution of silicone-modified pulullan, and TSPL-30-D5 which is a solicone solution, all available from Shin-Etsu Chemical Co., Ltd.

### (6) Antiperspirant

In one embodiment of the invention wherein the cosmetic composition is a deodorant, an antiperspirant is optionally blended therein. The antiperspirant is not particularly limited as long as it astricts the skin to suppress perspiration. Any of antiperspirants of general use may be used. Examples include aluminum chlorohydrate, aluminum chloride, aluminum chlorohydroxy allantoinate, aluminum allantoinate, tannic acid, aluminum potassium sulfate, zinc oxide, zinc p-phenolsulfonate, aluminum zirconium tetrachlorohydrate, and aluminum zirconium trichlorohydrex glycine. The preferred component which exerts a higher effect is an antiperspirant selected from aluminum halides, aluminum hydroxyhalides, and complexes or mixtures thereof with zirconium oxyhalides and zirconium hydroxyhalides. On use, these antiperspirants may be dissolved in water prior to blending or blended in powder form as such. Any of commercially available antiperspirants may be used. The commercially available product may take the form of a mixture with other components. The content of antiperspirant is not particularly limited and may be changed depending on the contents of other components. The content of antiperspirant is preferably 0.001 to 30% by weight, more preferably 0.01 to 20% by weight of the cosmetic composition for the purpose of obtaining a deodorant agent having a satisfactory antiperspirant effect or obtaining a deodorant agent which is less stimulative to the skin.

### (7) Antibacterial agent

The antibacterial agent is not particularly limited as long as it exerts a deodorant effect by controlling the propagation of indigenous bacteria on the skin which produce body odor-causing substances. Antibacterial agents including triclosan, benzalkonium chloride, benzethonium chloride, chlorhexidine hydrochloride, chlorhexidine gluconate, cloflucarban, and isomethylphenol are generally used. Any of extracts from green tea by dry distillation and essential oils or extracts from crude drugs may also be used as long as it has an antibacterial function. Examples of the antibacterial agent having a deodorant effect, included in the essential oils or extracts from crude drugs, include green tea extract, lavender extract, scutellaria baicalensis extract, Japanese goldthread extract, plantago asiatica extract, capillary wormwood extract, aloe extract, sophora angustifolia root extract, sasa veitchil leaf extract, garlic extract, witch-hazel extract, black tea extract, sage leaf extract, zanthoxylum piperitum peel extract, ginger root extract, acorus calamus rhizome extract, ivy extract, houttuynia cordata extract, peach fruit extract, peach leaf extract, peppermint leaf extract, cnidium officinale extract, eucalyptus leaf extract, peanut seedcoat extract, ganoderma lucidum extract, and sanguisorba officinalis root extract.

### (8) Other additives

Other additives include oil-soluble gelling agents, humectants, preservatives, fragrances, salts, antioxidants, acidity regulators, chelating agents, refreshing agents, anti-inflammatory agents, skin modifying ingredients (e.g., brightening or whitening agents, cell activators, anti-skin-roughening agents, blood flow enhancing agents, skin astringents, antiseborrheic agents), vitamins, amino acids, nucleic acids, hormones, and clathrate compounds.

### · Oil-soluble gelling agent

Exemplary oil-soluble gelling agents include organo-modified bentonite, metal soaps such as aluminum stearate, magnesium stearate, zinc myristate; amino acid derivatives such as N-lauroyl-L-glutamic acid and α,γ-di-n-butylamine; dextrin fatty acid esters such as dextrin palmitate, dextrin stearate, and dextrin 2-ethylhexanoate palmitate, sucrose fatty acid esters such as sucrose palmitate and sucrose stearate; fructooligosaccharide fatty acid esters such as fructooligosaccharide stearate and fructooligosaccharide 2-ethylhexanoate; and benzylidene derivatives of sorbitol such as monobenzylidene sorbitol and dibenzylidene sorbitol.

### · Humectant

Suitable humectants include glycerin, sorbitol, propylene glycol, dipropylene glycol, 1,3-butylene glycol, pentylene glycol, glucose, xylitol, maltitol, polyethylene glycol, hyaluronic acid, chondroitin sulfate, pyrrolidone carboxylate, polyoxyethylene methyl glucoside, polyoxypropylene methyl glucoside, yolk lecithin, soybean lecithin, phosphatidylcholine, phosphatidylethanol, phosphatidylserine, phosphatidylglycerol, phosphatidylinositol, and sphingolipid.

### · Preservative

Suitable preservatives include alkyl p-hydroxybenzoates, benzoic acid, sodium benzoate, sorbic acid, potassium sorbate, phenoxyethanol. Suitable antibacterial agents include benzoic acid, salicylic acid, phenol, sorbic acid, alkyl p-hydroxybenzoates, p-chloro-m-cresol, hexachlorophene, trichlorocarbanilide, and photosensitizers.

### · Fragrance

Fragrances include natural fragrances and synthetic fragrances. Suitable natural fragrances include plant fragrances separated from flowers, leaves, stems, and pericarps, and animal fragrances such as musk and civet. Suitable synthetic fragrances include hydrocarbons such as monoterpene; alcohols such as aliphatic alcohols and aromatic alcohols; aldehydes such as terpene aldehydes and aromatic aldehydes; ketones such as alicyclic ketones; esters such as terpene esters; lactones; phenols; oxides; nitrogen-containing compounds; and acetals.

### · Salt

Salts include inorganic salts, organic acid salts, amine salts, and amino acid salts. Exemplary inorganic salts include sodium, potassium, magnesium, calcium, aluminum, zirconium and zinc salts of inorganic acids such as hydrochloric acid, sulfuric acid, carbonic acid and nitric acid. Exemplary organic acid salts include salts of organic acids such as acetic acid, dehydroacetic acid, citric acid, malic acid, succinic acid, ascorbic acid, and stearic acid. Exemplary amine salts and amino acid salts include salts of amines such as triethanol amine, and salts of amino acids such as glutamic acid. Also useful are hyaluronic acid, salts such as chondroitin sulfate, aluminum zirconium glycine complex, and acid-alkali neutralized salts used in cosmetic formulation.

### · Antioxidant

Examples of the antioxidant include, but are not particularly limited to, carotenoid, ascorbic acid and salts thereof, ascorbyl stearate, tocopherol acetate, tocopherol, p-t-butylphenol, butyl hydroxyanisole, dibutyl hydroxytoluene, phytic acid, ferulic acid, thiotaurine, hypotaurine, sulfites, erythorbic acid and salts thereof, chlorogenic acid, epicatechin, epigallocatechin, epigallocatechin gallate, apigenin, kaempferol, myricetin, and quercetin.

### · Acidity regulator

Suitable acidity regulators include lactic acid, citric acid, glycolic acid, succinic acid, tartaric acid, dl-malic acid, potassium carbonate, sodium hydrogencarbonate, and ammonium hydrogencarbonate.

### · Chelating agent

Suitable chelating agents include alanine, sodium EDTA, sodium polyphosphate, sodium metaphosphate, phosphoric acid, aspartic acid diacetate, and ethylenediamine disuccinate.

### · Refreshing agent

Suitable refreshing agents include L-menthol and camphor.

### · Anti-inflammatory agent

Suitable anti-inflammatory agents include allantoin, glycyrrhizinic acid and salts thereof, glycyrrhetinic acid, stearyl glycyrrhetinate, tranexamic acid, and azulene.

### · Skin modifying ingredient

Suitable skin modifying ingredients include brightening or whitening agents such as vitamine C derivatives, hydroquinone, tranexamic acid, arbutin, phenyl ethyl resorcinol, kojic acid, plant extracts; cell activators such as royal jelly, photosensitizers, cholesterol derivatives, and bovine blood extracts; anti-skin-roughening agents; blood flow enhancing agents such as vanillylamide nonanoate, benzyl nicotinate, β-butoxyethyl nicotinate, capsaicin, zingerone, cantharides tincture, ichthammol, caffeine, tannic acid, α-borneol, tocopherol nicotinate, inositol hexanicotinate, cyclandelate, cinnarizine, tolazoline, acetylcholine, verapamil, cepharanthine, and γ-oryzanol; skin astringents such as zinc oxide and tannic acid; and antiseborrheic agents such as sulfur and thianthol.

### · Vitamin

Suitable vitamins include vitamin A family such as vitamin A oil, retinol, retinol acetate, retinol palmitate; vitamin B family, specifically vitamin B2 family such as riboflavin, riboflavin butyrate, and flavin adenine nucleotide, vitamin B6 family such as pyridoxine hydrochloride, pyridoxine dioctanoate, pyridoxine tripalmitate; vitamin B12 and derivatives thereof, vitamin B15 and derivatives thereof; vitamin C family such as L-ascorbic acid, L-ascorbic acid dipalmitic acid ester, L-ascorbic acid-2-sodium sulfate, and dipotassium L-ascorbic acid phosphoric acid diester; vitamin D family such as ergocalciferol and cholecalciferol; vitamin E family such as α-tocopherol, β-tocopherol, γ-tocopherol, dl-α-tocopherol acetate, dl-α-tocopherol nicotinate, and dl-α-tocopherol succinate; nicotinic acids such as nicotinic acid, benzyl nicotinate, and nicotinic amide; vitamin H, vitamin P, pantothenic acids such as calcium pantothenate, D-pantothenyl alcohol, pantothenyl ethyl ether, acetylpantothenyl ethyl ether; and biotin.

### · Amino acid

Suitable amino acids include glycine, valine, leucine, isoleucine, serine, threonine, phenylalanine, arginine, lysine, aspartic acid, glutamic acid, cystine, cysteine, methionine, and tryptophan.

### · Nucleic acid

Typical of the nucleic acid is deoxyribonucleic acid.

### · Hormone

Suitable hormones include estradiol and ethenyl estradiol.

### · Clathrate compound

Typical of the clathrate compound is cyclodextrin.

The inventive cosmetic composition may take a variety of formulas including liquid, multilayer, milk, cream, stick, solid, moose, powder, and spray. Exemplary types of the cosmetic composition include make-up cosmetic products such as skin care, hair care, cleansing, make-up base, concealer, liquid foundation, cream foundation, solid foundation, cheek color, eye shadow, mascara, eye liner, eye brow, and lipstick, and UV-protecting cosmetic products such as sun care emulsions and creams containing hydrophobized coloring pigments. The cosmetic composition is particularly suited as sun care products because of a low viscosity, pleasant feeling, and high UV-shielding effect.

When the dispersion is prepared according to the invention, the method and apparatus are not particularly limited and any well-known method may be used. For example, any of agitators, grinders, mixers, and dispersing machines such as Henschel mixers, ball mills, kneaders, planetary mixers, ribbon blenders, dispersing mixers, homomixers, roll mills, and bead mills may be used. In particular, dispersion on a bead mill is preferred from the aspect of mixing efficiency.

The cosmetic composition may take the form of either an emulsified composition or a non-aqueous composition. An emulsified composition is selected when it is desired to impart a fresh feeling on use. The emulsion form may be either an O/W emulsion or a W/O emulsion while even an O/O composition is acceptable. As used herein, the "non-aqueous composition" refers to an oily composition substantially free of water. The method of preparing the cosmetic composition is not particularly limited and the cosmetic composition may be prepared by any well-known method.

### EXAMPLES

Examples and Comparative Examples are shown below by way of illustration and not by way of limitation. In Examples, compositional percent (%) is by weight unless otherwise stated. The viscosity of a composition is as measured at 25°C by a rotational viscometer according to JIS K 7117-1: 1999. The kinematic viscosity of component (D) is as measured at 25°C by a Cannon-Fenske viscometer according to JIS Z 8803: 2011.

### [Synthesis Example 1]

A reactor was charged with 70 g of methylhydrogenpolysiloxane having the formula (21), 69 g of 1-dodecene, and 21 mg of a 3% ethanol solution of chloroplatinic acid. Reaction was carried out at 80°C for 1 hour. It is noted that the arrangement order of siloxane units is not limited to the designated one (the same holds true, hereinafter).

Then, 32.9 g of polyglycerin allyl ether having the formula (22), 70 g of isopropyl alcohol (IPA), and 15 mg of a 3% ethanol solution of chloroplatinic acid were added to the reaction solution, which was held at 80°C for 2 hours for reaction.

At the end of reaction, 130 g of organopolysiloxane having the formula (23) and 15 mg of a 3% ethanol solution of chloroplatinic acid were added to the reaction solution, after which reaction was carried out at 80°C for 3 hours.

After the reaction solution was cooled at 50°C, 40 g of 0.005N (mol/L) HCl aqueous solution was added to the reaction solution, which was stirred for 2 hours to carry out hydrolysis of unreacted allyl ether groups in formula (22). After the reaction solution was cooled at 25°C, 0.34 g of 5% sodium hydrogencarbonate aqueous solution was added and thoroughly stirred for neutralization. The solvent was removed by vacuum distillation (130°C/5 mmHg). Thereafter, 110 g of methanol was added to the residue and thoroughly stirred. The mixture was allowed to stand for 2 hours for extracting and removing the unreacted polyglycerin having formula (22). The solvent was removed by vacuum distillation (130°C/5 mmHg). The salt precipitate was filtered, obtaining an organopolysiloxane having formula (I) wherein a+b+c+d=7.5, (b+c)/(a+d)=1.9, total weight of R² is 24% by weight of one molecule, and total weight of R³ is 11% by weight of one molecule. This product was a pale yellow transparent liquid having a viscosity of 200 mPa·s.

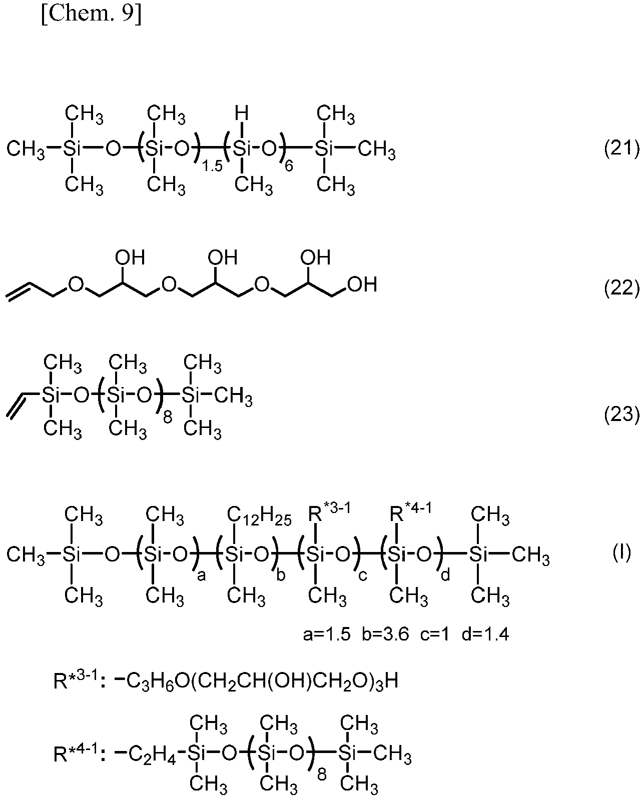

### [Synthesis Example 2]

A reactor was charged with 70 g of methylhydrogenpolysiloxane having the formula (24), 44 g of 1-dodecene, and 15 mg of a 3% ethanol solution of chloroplatinic acid. Reaction was carried out at 80°C for 1 hour.

Then, 19.4 g of polyglycerin allyl ether having the formula (25), 60 g of IPA, and 15 mg of a 3% ethanol solution of chloroplatinic acid were added to the reaction solution, which was held at 80°C for 2 hours for reaction.

At the end of reaction, organopolysiloxane having the formula (26) and 15 mg of a 3% ethanol solution of chloroplatinic acid were added to the reaction solution, after which reaction was carried out at 80°C for 3 hours.

After the reaction solution was cooled at 50°C, 40 g of 0.005N (mol/L) HCl aqueous solution was added to the reaction solution, which was stirred for 2 hours to carry out hydrolysis of unreacted allyl ether groups in formula (25). After the reaction solution was cooled at 25°C, 0.34 g of 5% sodium hydrogencarbonate aqueous solution was added and thoroughly stirred for neutralization. The solvent was removed by vacuum distillation (130°C/5 mmHg). Thereafter, 65 g of methanol was added to the residue and thoroughly stirred. The mixture was allowed to stand for 2 hours for extracting and removing the unreacted polyglycerin having formula (25). The solvent was removed by vacuum distillation (130°C/5 mmHg). The salt precipitate was filtered, obtaining an organopolysiloxane having formula (II) wherein a+b+c+d=15.8, (b+c)/(a+d)=0.6, total weight of R² is 26% by weight of one molecule, and total weight of R³ is 14% by weight of one molecule. This product was a colorless transparent liquid having a viscosity of 2,700 mPa·s.

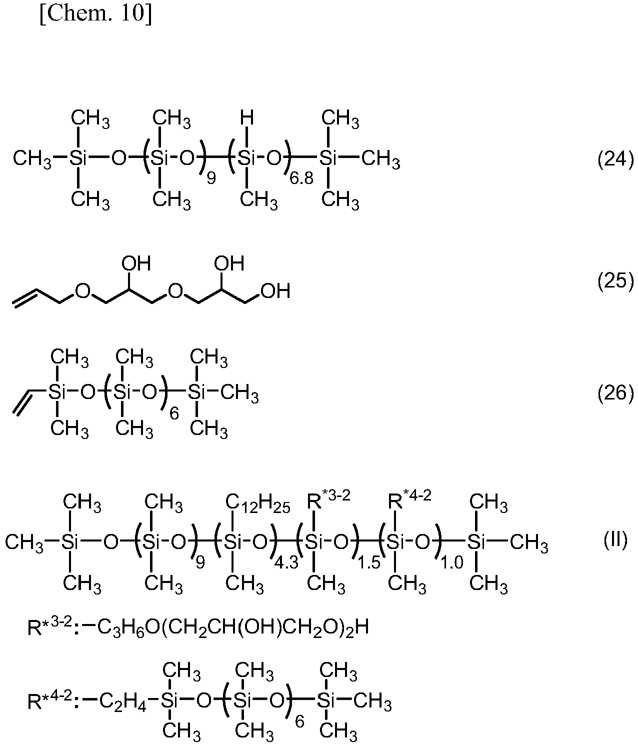

### [Synthesis Example 3]

A reactor was charged with 113 g of methylhydrogenpolysiloxane having the formula (27), 34 g of 1-dodecene, and 15 mg of a 3% ethanol solution of chloroplatinic acid. Reaction was carried out at 80°C for 1 hour.

Then, 43 g of polyglycerin allyl ether having the formula (22), 74 g of IPA, and 21 mg of a 3% ethanol solution of chloroplatinic acid were added to the reaction solution, which was held at 80°C for 2 hours for reaction.

At the end of reaction, organopolysiloxane having the formula (28) and 21 mg of a 3% ethanol solution of chloroplatinic acid were added to the reaction solution, after which reaction was carried out at 80°C for 3 hours.

After the reaction solution was cooled at 50°C, 30 g of 0.005N (mol/L) HCl aqueous solution was added to the reaction solution, which was stirred for 2 hours to carry out hydrolysis of unreacted allyl ether groups in formula (22). After the reaction solution was cooled at 25°C, 0.25 g of 5% sodium hydrogencarbonate aqueous solution was added and thoroughly stirred for neutralization. The solvent was removed by vacuum distillation (130°C/5 mmHg). Thereafter, 90 g of methanol was added to the residue and thoroughly stirred. The mixture was allowed to stand for 2 hours for extracting and removing the unreacted polyglycerin having formula (22). The solvent was removed by vacuum distillation (130°C/5 mmHg). The salt precipitate was filtered, obtaining an organopolysiloxane having formula (III) wherein a+b+c+d=16.6, (b+c)/(a+d)=1.2, total weight of R² is 35% by weight of one molecule, and total weight of R³ is 17% by weight of one molecule. This product was a pale yellow transparent liquid having a viscosity of 2,680 mPa·s.

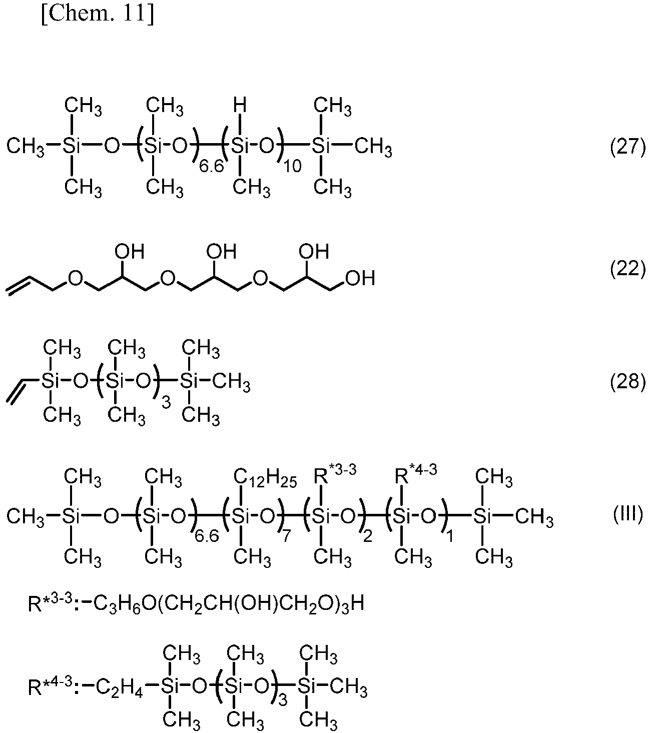

### [Synthesis Example 4]

A reactor was charged with 67 g of methylhydrogenpolysiloxane having the formula (29), 54 g of 1-dodecene, and 15 mg of a 3% ethanol solution of chloroplatinic acid. Reaction was carried out at 80°C for 1 hour.

Then, 24 g of polyglycerin allyl ether having the formula (22), 65 g of IPA, and 21 mg of a 3% ethanol solution of chloroplatinic acid were added to the reaction solution, which was held at 80°C for 2 hours for reaction.

At the end of reaction, 72 g of organosiloxane having the formula (23) and 21 mg of a 3% ethanol solution of chloroplatinic acid were added to the reaction solution, after which reaction was carried out at 80°C for 3 hours.

After the reaction solution was cooled at 50°C, 30 g of 0.005N (mol/L) HCl aqueous solution was added to the reaction solution, which was stirred for 2 hours to carry out hydrolysis of unreacted allyl ether groups in formula (22). After the reaction solution was cooled at 25°C, 0.26 g of 5% sodium hydrogencarbonate aqueous solution was added and thoroughly stirred for neutralization. The solvent was removed by vacuum distillation (130°C/5 mmHg). Thereafter, 120 g of methanol was added to the residue and thoroughly stirred. The mixture was allowed to stand for 2 hours for extracting and removing the unreacted polyglycerin having formula (22). The solvent was removed by vacuum distillation (130°C/5 mmHg). The salt precipitate was filtered, obtaining an organopolysiloxane having formula (IV) wherein a+b+c+d=12.6, (b+c)/(a+d)=0.6, total weight of R² is 25% by weight of one molecule, and total weight of R³ is 11% by weight of one molecule. This product was a pale yellow transparent liquid having a viscosity of 840 mPa-s.

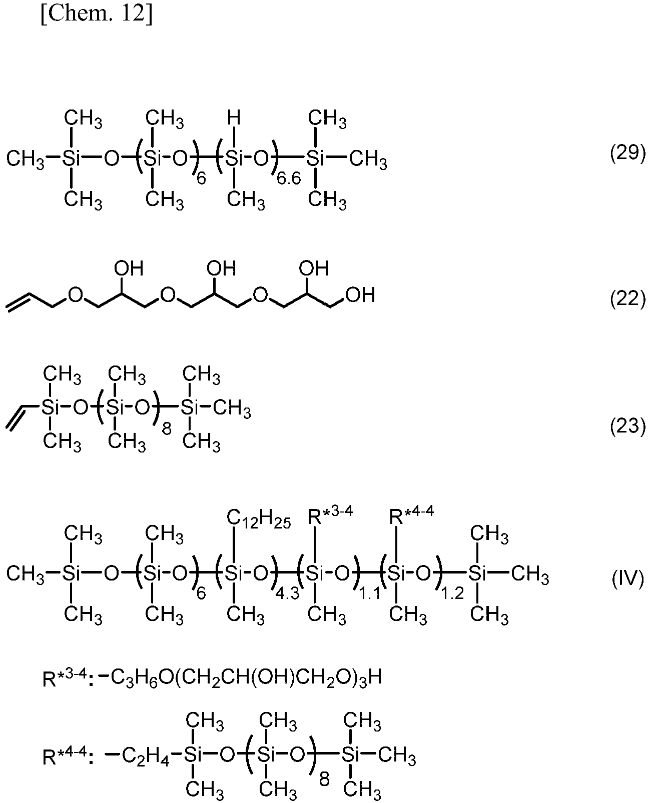

### [Synthesis Example 5]

A reactor was charged with 1,438 g of methylhydrogenpolysiloxane having the formula (31), 997 g of 1-hexadecene, and 150 mg of a 3% ethanol solution of chloroplatinic acid. Reaction was carried out at 80°C for 1 hour.

Then, 420 g of polyglycerin allyl ether having the formula (22), 1,223 g of IPA, and 150 mg of a 3% ethanol solution of chloroplatinic acid were added to the reaction solution, which was held at 80°C for 2 hours for reaction.

At the end of reaction, 985 g of organosiloxane having the formula (23) and 150 mg of a 3% ethanol solution of chloroplatinic acid were added to the reaction solution, after which reaction was carried out at 80°C for 3 hours.

After the reaction solution was cooled at 50°C, 635 g of 0.005N (mol/L) HCl aqueous solution was added to the reaction solution, which was stirred for 2 hours to carry out hydrolysis of unreacted allyl ether groups. After the reaction solution was cooled, 5.5 g of 5% sodium hydrogencarbonate aqueous solution was added and thoroughly stirred for neutralization. The solvent was removed by vacuum distillation (130°C/5 mmHg). Thereafter, 2,000 g of methanol was added to the residue and thoroughly stirred. The mixture was allowed to stand for 2 hours for extracting and removing the unreacted polyglycerin. The solvent was removed by vacuum distillation (130°C/5 mmHg). The salt precipitate was filtered, obtaining an organopolysiloxane having the following formula (V) wherein a+b+c+d=16.7, (b+c)/(a+d)=0.7, total weight of R² is 36% by weight of one molecule, and total weight of R³ is 11% by weight of one molecule. This product was a pale yellow transparent liquid having a viscosity of 1,930 mPa·s.

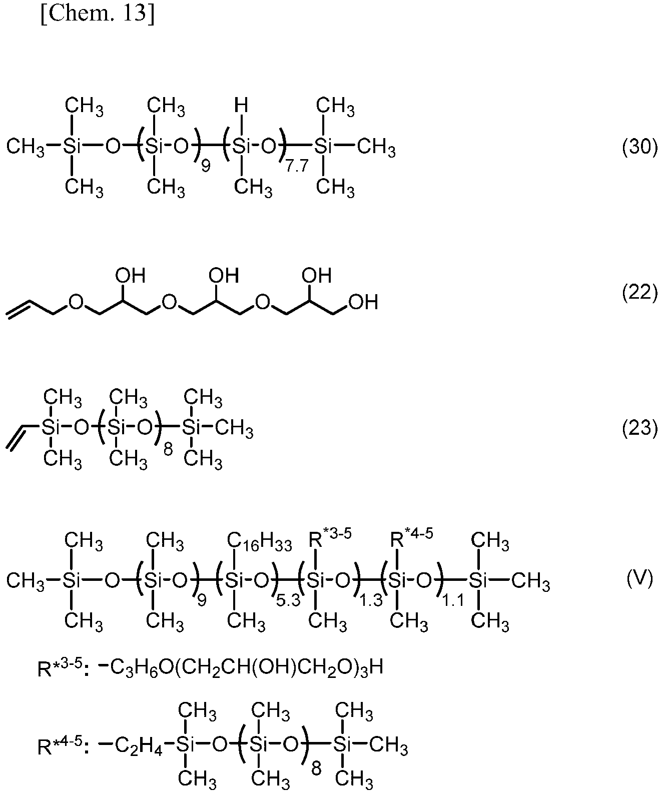

### [Synthesis Example 6]

A reactor was charged with 956 g of methylhydrogenpolysiloxane having the formula (31), 1,014 g of 1-dodecene, and 150 mg of a 3% ethanol solution of chloroplatinic acid. Reaction was carried out at 80°C for 1 hour.

Then, 281 g of polyglycerin allyl ether having the formula (22), 1,223 g of IPA, and 150 mg of a 3% ethanol solution of chloroplatinic acid were added to the reaction solution, which was held at 80°C for 2 hours for reaction.

At the end of reaction, 475 g of organosiloxane having the formula (23) and 150 mg of a 3% ethanol solution of chloroplatinic acid were added to the reaction solution, after which reaction was carried out at 80°C for 3 hours.

After the reaction solution was cooled at 50°C, 40 g of 0.005N (mol/L) HCl aqueous solution was added to the reaction solution, which was stirred for 2 hours to carry out hydrolysis of unreacted allyl ether groups. After the reaction solution was cooled, 3.4 g of 5% sodium hydrogencarbonate aqueous solution was added and thoroughly stirred for neutralization. The solvent was removed by vacuum distillation (130°C/5 mmHg). Thereafter, 1,600 g of methanol was added to the residue and thoroughly stirred. The mixture was allowed to stand for 2 hours for extracting and removing the unreacted polyglycerin. The solvent was removed by vacuum distillation (130°C/5 mmHg). The salt precipitate was filtered, obtaining an organopolysiloxane having formula (VI) wherein a+b+c+d=11.4, (b+c)/(a+d)=1.6, total weight of R² is 37% by weight of one molecule, and total weight of R³ is 10% by weight of one molecule. This product was a pale yellow transparent liquid having a viscosity of 720 mPa·s.

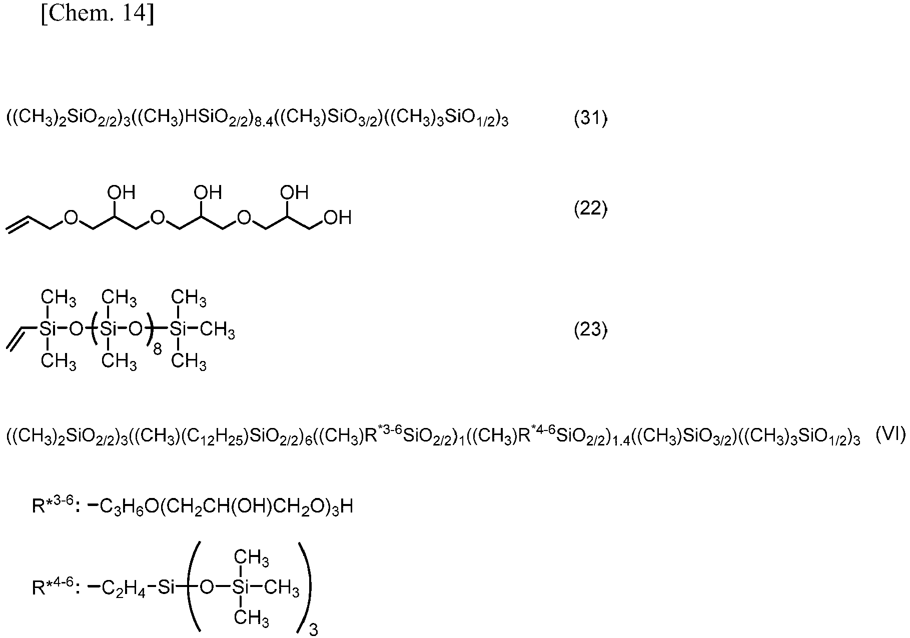

### [Comparative Synthesis Examples 1 to 9]

Organopolysiloxanes of Comparative Synthesis Examples 1 to 9 shown in the following Table were synthesized by the same procedure as in Synthesis Example 1 except that the weight ratio of reactants in Synthesis Example 1 was changed. The ratio and physical properties of the reactants are shown in Table 1. In the Table, a, b, c and d indicate the number of corresponding repeat units in the following formula.

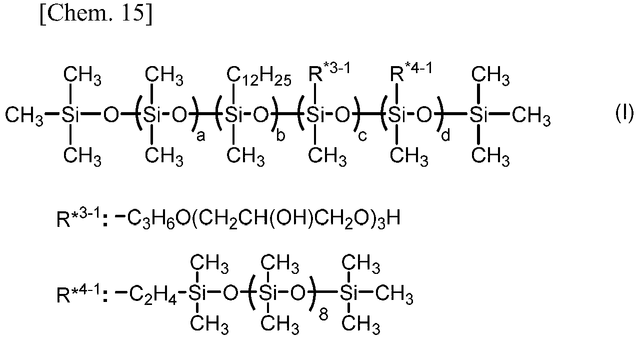

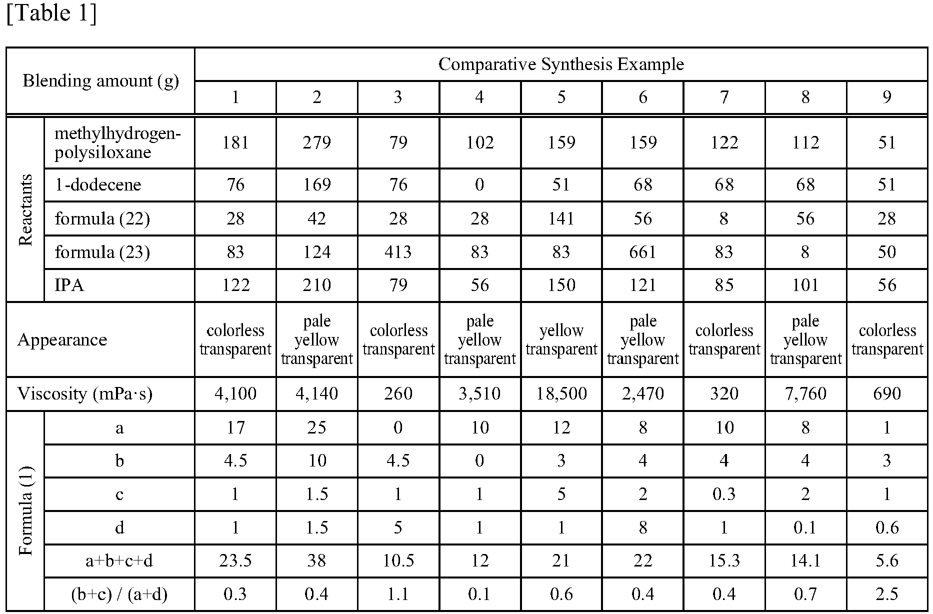

### [Examples 1 to 12 and Comparative Examples 1 to 18]

Using each of the organopolysiloxanes obtained in Synthesis Examples and Comparative Synthesis Examples, a dispersion or slurry was prepared on a bead mill in accordance with the formulation shown in Tables 2 to 4. In Tables, the term "Synthesis Example 1" means the organopolysiloxane obtained in Synthesis Example 1. The dispersions were evaluated for dispersibility as follows. The results are also shown in the Tables.

### [Evaluation of dispersion viscosity (dispersibility)]

In a glass bottle, 100 g of the dispersion was stored at 25°C for 2 months. The viscosity of the slurry immediately after preparation and after 2 months was measured at 25°C by a B-type viscometer (Model TVB-10 by Toki Sangyo Co., Ltd.) according to the method of JIS K 7117-1: 1999. The results are rated according to the following evaluation criterion.

### [Evaluation criterion]

⊚: from 1,000 mPa·s to less than 2,000 mPa·s
○: from 2,000 mPa-s to less than 4,000 mPa·s
△: from 4,000 mPa·s to less than 10,000 mPa·s
×: 10,000 mPa·s or more, not flowable

**[Table 2]**

| Composition (%) | | Example | | | | | | | | | | | |
|---|---|---|---|---|---|---|---|---|---|---|---|---|---|
| | | 1 | 2 | 3 | 4 | 5 | 6 | 7 | 8 | 9 | 10 | 11 | 12 |
| (A) | Synthesis Example 1 | 10 | | | | | | 5 | | | | | |
| | Synthesis Example 2 | | 10 | | | | | | 5 | | | | |
| | Synthesis Example 3 | | | 10 | | | | | | 5 | | | |
| | Synthesis Example 4 | | | | 10 | | | | | | 5 | | |
| | Synthesis Example 5 | | | | | 10 | | | | | | 5 | |
| | Synthesis Example 6 | | | | | | 10 | | | | | | 5 |
| (C) STR-100A-LP *1 | | 50 | 50 | 50 | 50 | 50 | 50 | | | | | | |
| (C) FINEX-33W-LP2 *2 | | | | | | | | 65 | 65 | 65 | 65 | 65 | 65 |
| (D) cyclopentasiloxane | | 40 | 40 | 40 | 40 | 40 | 40 | 30 | 30 | 30 | 30 | 30 | 30 |
| Dispersion viscosity | immediately after preparation | ○ | 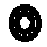 | ○ | 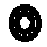 | 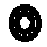 | ○ | 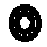 | 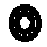 | 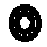 | 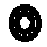 | 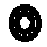 | 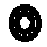 |
| | after 2 months | ○ | 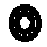 | ○ | 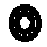 | 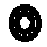 | ○ | ○ | ○ | 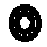 | 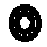 | 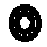 | ○ |

**[Table 3]**

| Composition (%) | | Comparative Example | | | | | | | | |
|---|---|---|---|---|---|---|---|---|---|---|
| | | 1 | 2 | 3 | 4 | 5 | 6 | 7 | 8 | 9 |
| (A) Comparison | Comparative Synthesis Example 1 | 10 | | | | | | | | |
| | Comparative Synthesis Example 2 | | 10 | | | | | | | |
| | Comparative Synthesis Example 3 | | | 10 | | | | | | |
| | Comparative Synthesis Example 4 | | | | 10 | | | | | |
| | Comparative Synthesis Example 5 | | | | | 10 | | | | |
| | Comparative Synthesis Example 6 | | | | | | 10 | | | |
| | Comparative Synthesis Example 7 | | | | | | | 10 | | |
| | Comparative Synthesis Example 8 | | | | | | | | 10 | |
| | Comparative Synthesis Example 9 | | | | | | | | | 10 |
| (C) STR-100A-LP *1 | | 50 | 50 | 50 | 50 | 50 | 50 | 50 | 50 | 50 |
| (D) cyclopentasiloxane | | 40 | 40 | 40 | 40 | 40 | 40 | 40 | 40 | 40 |
| Dispersion viscosity | immediately after preparation | ○ | Δ | × | Δ | × | Δ | × | × | Δ |
| | after 2 months | Δ | × | × | × | × | × | × | × | × |

**[Table 4]**

| Composition (%) | | Comparative Example | | | | | | | | |
|---|---|---|---|---|---|---|---|---|---|---|
| | | 10 | 11 | 12 | 13 | 14 | 15 | 16 | 17 | 18 |
| (A) Comparison | Comparative Synthesis Example 1 | 5 | | | | | | | | |
| | Comparative Synthesis Example 2 | | 5 | | | | | | | |
| | Comparative Synthesis Example 3 | | | 5 | | | | | | |
| | Comparative Synthesis Example 4 | | | | 5 | | | | | |
| | Comparative Synthesis Example 5 | | | | | 5 | | | | |
| | Comparative Synthesis Example 6 | | | | | | 5 | | | |
| | Comparative Synthesis Example 7 | | | | | | | 5 | | |
| | Comparative Synthesis Example 8 | | | | | | | | 5 | |
| | Comparative Synthesis Example 9 | | | | | | | | | 5 |
| (C) FINEX-33W-LP2 *2 | | 65 | 65 | 65 | 65 | 65 | 65 | 65 | 65 | 65 |
| (D) cyclopentasiloxane | | 30 | 30 | 30 | 30 | 30 | 30 | 30 | 30 | 30 |
| Dispersion viscosity | immediately after preparation | Δ | Δ | Δ | Δ | × | × | Δ | × | Δ |
| | after 2 months | Δ | × | Δ | × | × | × | × | × | × |

The components used in Tables 2 to 4 are identified below.
(*1) hydrous silica (labeling name, hydrated silica)/Al hydroxide (labeling name, aluminum hydroxide)/hydrogen dimethicone (labeling name, hydrogen dimethicone)-treated titanium oxide (labeling name, titanium dioxide)
(*2) hydrous silica (labeling name, hydrated silica)/hydrogen dimethicone (labeling name, hydrogen dimethicone)-treated zinc oxide (labeling name, zinc oxide)
kinematic viscosity of (D) cyclopentasiloxane: 4.0 mm²/s

The blending amounts in Tables are amounts of components blended (the same holds true, hereinafter).

As is evident from the results, all the dispersions of Examples 1 to 12 had a low viscosity and satisfactory stability with time. In contrast, the dispersions of Comparative Examples 1 to 18 had a high viscosity immediately after preparation or showed an outstanding viscosity buildup with the lapse of time despite a low initial viscosity. It is thus demonstrated that the organopolysiloxane within the scope of the invention has a high dispersibility and provides a dispersion having a low viscosity and better storage stability.

When an organopolysiloxane had a long backbone like the organopolysiloxanes obtained in Comparative Synthesis Examples 1 and 2, a low viscosity dispersion was not obtained.

When an organopolysiloxane was free of dimethylsiloxane units like the organopolysiloxane obtained in Comparative Synthesis Example 3, or when an organopolysiloxane was free of alkyl branches like the organopolysiloxane obtained in Comparative Synthesis Example 4, a low viscosity dispersion was not obtained.

When an organopolysiloxane had an excess of polyglycerin groups or silicone branches introduced therein like the organopolysiloxanes obtained in Comparative Synthesis Examples 5 and 6, a low viscosity dispersion was not obtained.

When an organopolysiloxane had extremely less polyglycerin groups or silicone branches like the organopolysiloxanes obtained in Comparative Synthesis Examples 7 and 8, a low viscosity dispersion was not obtained.

### [Examples 13, 14 and Comparative Examples 19 to 24]

Cosmetic compositions in the form of W/O type cream were prepared in accordance with the formulation shown in Table 5. The cosmetic compositions were evaluated by the following tests, with the results shown in Table 5.

### (Preparation method)

Components 1 to 10 were mixed until uniform. After uniform mixing, components 11 to 13 were slowly added to the mix and agitated to form an emulsion. A predetermined vessel was filled with the emulsion, obtaining a cosmetic composition in the form of W/O type cream.

The cosmetic composition was evaluated in terms of (1) spread (or extensibility), (2) skin penetration, and (3) stability according to the following judgment criteria.

### [Evaluation of properties]

The cosmetic composition was evaluated in terms of spread and skin penetration according to the following evaluation criterion by a panel of ten members. The result is an average of ratings of 10 panel members and rated according to the following judgment criterion.

### [Evaluation criterion]

### Points

5: excellent
4: good
3: mediocre
2: rather poor
1: poor

After the average point was computed, the sample was rated in the "○-× scale" according to the following criterion.

### [Evaluation criterion]

### Average point

⊚: 4.5 or more
○: from 3.5 to less than 4.5
△: from 2.5 to less than 3.5
×: from 1.5 to less than 2.5
××: less than 1.5

A sample is judged Pass for "O" or above.

### [Stability with time]

A 30-mL vial was filled with the cosmetic composition and stored in a thermostatic chamber at 50°C for 1 week, after which the viscosity of the composition was measured. In terms of a change of viscosity from that immediately after preparation, the rating is excellent "⊚" for a change of 0% to less than 25%, good "○" for a change of 25% to less than 40%, fair "△" for a change of 40% to less than 50%, and poor "×" for a change of 50% or more or separated. The composition which was rated poor "×" in stability was no longer evaluated for properties. The viscosity of the compositions of Examples and Comparative Examples immediately after preparation was in the range of 10,000 to 200,000 mPa·s when they were viscous.

The viscosity was measured by a B-type viscometer (Model TVB-10 by Toki Sangyo Co., Ltd.) with spindle No. 4 at a rotational speed of 6 rpm and a time of 60 seconds.

A sample is judged Pass for "O" or above.

**[Table 5]**

| Formulation (%) | | Example | | Comparative Example | | | | | |
|---|---|---|---|---|---|---|---|---|---|
| | | 13 | 14 | 19 | 20 | 21 | 22 | 23 | 24 |
| 1 | (A) Synthesis Example 4 | 2.0 | | | | | | | |
| 2 | (A) Synthesis Example 5 | | 2.0 | | | | | | |
| 3 | Comparative Synthesis Example 1 | | | 2.0 | | | | | |
| 4 | Comparative Synthesis Example 2 | | | | 2.0 | | | | |
| 5 | Comparative Synthesis Example 3 | | | | | 2.0 | | | |
| 6 | Comparative Synthesis Example 4 | | | | | | 2.0 | | |
| 7 | Comparative Synthesis Example 5 | | | | | | | 2.0 | |
| 8 | Comparative Synthesis Example 9 | | | | | | | | 2.0 |
| 9 | (D) KF-56A (*1) | 13.8 | 13.8 | 13.8 | 13.8 | 13.8 | 13.8 | 13.8 | 13.8 |
| 10 | (B) ethylhexyl methoxycinnamate | 9.2 | 9.2 | 9.2 | 9.2 | 9.2 | 9.2 | 9.2 | 9.2 |
| 11 | BG | 5.0 | 5.0 | 5.0 | 5.0 | 5.0 | 5.0 | 5.0 | 5.0 |
| 12 | sodium chloride | 0.5 | 0.5 | 0.5 | 0.5 | 0.5 | 0.5 | 0.5 | 0.5 |
| 13 | Water | balance | balance | balance | balance | balance | balance | balance | balance |
| Total | | 100.0 | 100.0 | 100.0 | 100.0 | 100.0 | 100.0 | 100.0 | 100.0 |
| Spread | | 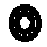 | ○ | Δ | Δ | ○ | 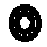 | Δ | ○ |
| Skin penetration | | 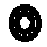 | 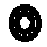 | × | Δ | ○ | Δ | ○ | 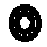 |
| Stability | | 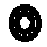 | 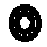 | ○ | 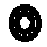 | Δ | × | Δ | Δ |

| | | | | | | | | | |
|---|---|---|---|---|---|---|---|---|---|
| (*1) diphenylsiloxyphenyl trimethicone (labeling name, INCI: Diphenylsiloxyphenyl Trimethicone), kinematic viscosity 15 mm²/s | | | | | | | | | |

As is evident from the above results, the cosmetic compositions in the form of W/O cream of Examples 11 and 12 demonstrate excellent stability while maintaining good spread and skin penetration, as compared with Comparative Examples 19 to 24.

### [Example 15]

An eye cream was prepared according to the formulation in Table 6.

**[Table 6]**

| Formulation (%) | | Example 15 |
|---|---|---|
| 1 | Crosslinked dimethylpolysiloxane mixture (*1) | 35.0 |
| 2 | Dimethicone | 10.0 |
| 3 | 1,3-butylene glycol | 3.0 |
| 4 | Polyglycerin-modified silicone (*2) | 0.6 |
| 5 | Synthesis Example 4 | 0.3 |
| 6 | (Ammonium acryloyldimethyltaurine/VP) copolymer | 0.7 |
| 7 | (Na acrylate/Na acryloyldimethyltaurine) copolymer | 0.6 |
| 8 | Sodium chloride | 0.2 |
| 9 | Purified water | balance |
| Total | | 100.0 |

| | | |
|---|---|---|
| (*1) KSG-15 from Shin-Etsu Chemical Co., Ltd. (mixture of decamethylcyclopentasiloxane 90-96% and crosslinked dimethylpolysiloxane 4-10%) (*2) KF-6100 from Shin-Etsu Chemical Co., Ltd. (polyglyceryl-3 disiloxane dimethicone (labeling name, INCI: Polyglyceryl-3 Disiloxane Dimethicone)) | | |

### (Preparation method)

Components 3 to 9 were mixed until uniform. After uniform mixing, components 1 and 2 were slowly added to the mix and agitated to form an emulsion. A predetermined vessel was filled with the emulsion, obtaining an eye cream. The resulting eye cream spread lightly, was dry and non-sticky, and presented a pleasant feeling on use.

### [Example 16]

A sun care emulsion was prepared according to the formulation in Table 7.

**[Table 7]**

| Formulation (%) | | Example 16 |
|---|---|---|
| 1 | Crosslinked polyether-modified silicone mixture (*1) | 3.0 |
| 2 | Synthesis Example 4 | 3.0 |
| 3 | Ethylhexyl methoxycinnamate | 7.0 |
| 4 | Dimethicone (6 cs) | 20.0 |
| 5 | Diphenylsiloxyphenyl trimethicone (*2) | 5.0 |
| 6 | Microparticulate titanium oxide (*3) | 5.0 |
| 7 | Microparticulate zinc oxide (*4) | 5.0 |
| 8 | 1,3-butylene glycol | 5.0 |
| 9 | Sodium chloride | 0.5 |
| 10 | Sodium citrate | 0.2 |
| 11 | Purified water | balance |
| Total | | 100.0 |

| | | |
|---|---|---|
| (*1) KSG-210 from Shin-Etsu Chemical Co., Ltd. (mixture of dimethylpolysiloxane 70-80% and crosslinked polyether-modified silicone 20-30%) (*2) KF-56A from Shin-Etsu Chemical Co., Ltd. (*3) MT-150EX from Tayca Corp. (*4) MZX-5080TS from Tayca Corp. | | |

### (Preparation method)

On a dispersing machine (Homodisper), components 1 to 7 were thoroughly mixed until uniform. After uniform mixing, components 8 to 11 were slowly added to the mix and agitated to form an emulsion. A predetermined vessel was filled with the emulsion, obtaining a sun care emulsion. The resulting emulsion spread lightly, was dry and non-sticky, and presented a pleasant feeling on use.

### [Example 17]

A cosmetic base was prepared according to the formulation in Table 8.

**[Table 8]**

| Formulation (%) | | Example 17 |
|---|---|---|
| 1 | Crosslinked polyether-modified silicone mixture (*1) | 3.0 |
| 2 | Crosslinked dimethylpolysiloxane mixture (*2) | 1.0 |
| 3 | Synthesis Example 4 | 0.5 |
| 4 | Dimethicone (6 cs) | 8.4 |
| 5 | Spherical silicone composite powder (*3) | 2.0 |
| 6 | Dispersion of Example 4 | 20.0 |
| 7 | 1,3-butylene glycol | 8.0 |
| 8 | Ethanol | 5.0 |
| 9 | Sodium chloride | 0.5 |
| 10 | Sodium citrate | 0.2 |
| 11 | Water | balance |
| Total | | 100.0 |

| | | |
|---|---|---|
| (*1) KSG-210 from Shin-Etsu Chemical Co., Ltd. (mixture of dimethylpolysiloxane 70-80% and crosslinked polyether-modified silicone 20-30%) (*2) KSG-15 from Shin-Etsu Chemical Co., Ltd. (mixture of decamethylpentasiloxane 90-96% and crosslinked dimethylpolysiloxane 4-10%) (*3) KSP-105 from Shin-Etsu Chemical Co., Ltd. | | |

### (Preparation method)

Components 1 to 5 were mixed until uniform. After uniform mixing, components 7 to 11 were slowly added to the mix and agitated to form an emulsion. Component 6 was added and agitated until uniform. A predetermined vessel was filled with the emulsion, obtaining a cosmetic base. The resulting base spread lightly, was dry and non-sticky, and presented a pleasant feeling on use.

### [Example 18]

A sun care cream was prepared according to the formulation in Table 9.

**[Table 9]**

| Formulation (%) | | Example 18 |
|---|---|---|
| 1 | Crosslinked polyether-modified silicone mixture (*1) | 3.0 |
| 2 | Crosslinked dimethylpolysiloxane mixture (*2) | 3.0 |
| 3 | Synthesis Example 4 | 3.0 |
| 4 | Decamethylcyclopentasiloxane | 2.0 |
| 5 | Dimethicone (6 cs) | 3.0 |
| 6 | Isononyl isononanoate | 4.8 |
| 7 | Spherical silicone composite powder (*3) | 1.0 |
| 8 | Dispersion of Example 4 | 31.2 |
| 9 | Dispersion of Example 9 | 35.0 |
| 10 | 1,3-butylene glycol | 2.0 |
| 11 | Sodium chloride | 0.5 |
| 12 | Sodium citrate | 0.2 |
| 13 | Purified water | balance |
| Total | | 100.0 |

| | | |
|---|---|---|
| (*1) KSG-210 from Shin-Etsu Chemical Co., Ltd. (mixture of dimethylpolysiloxane 70-80% and crosslinked polyether-modified silicone 20-30%) (*2) KSG-15 from Shin-Etsu Chemical Co., Ltd. (mixture of decamethylcyclopentasiloxane 90-96% and crosslinked dimethylpolysiloxane 4-10%) (*3) KSP-105 from Shin-Etsu Chemical Co., Ltd. | | |

### (Preparation method)

Components 1 to 7 were mixed until uniform. After uniform mixing, components 10 to 13 were slowly added to the mix and agitated to form an emulsion. Components 8 and 9 were added and agitated until uniform. A predetermined vessel was filled with the emulsion, obtaining a sun care cream. The resulting cream spread lightly, was dry and non-sticky, and presented a pleasant feeling on use.

### [Examples 19 to 32 and Comparative Examples 25, 26]

Using a paint shaker (media diameter 1.5 mm), a slurry was prepared according to the formulation in Table 10.

**[Table 10]**

| Formulation (%) | Example | | | | | | | |
|---|---|---|---|---|---|---|---|---|
| | 19 | 20 | 21 | 22 | 23 | 24 | 25 | 26 |
| Synthesis Example 4 | 10 | 10 | 10 | 10 | 10 | 10 | 10 | 10 |
| Comparative Synthesis Example 2 | - | - | - | - | - | - | - | - |
| MT-100TV (*1) | 40 | 40 | 40 | 35 | 35 | 40 | 40 | - |
| MZ-306X (*2) | - | - | - | - | - | - | - | 50 |
| KF-56A (*3) | - | 12 | - | balance | - | - | - | - |
| Ethylhexyl methoxycinnamate | - | 8 | - | - | - | - | - | - |
| Dimethicone (2 cs) | balance | balance | - | - | - | - | - | balance |
| Dodecane | - | - | balance | - | - | - | - | - |
| Ethylhexyl palmitate | - | - | - | - | balance | - | - | - |
| Ethylmethicone | - | - | - | - | - | balance | - | - |
| Ethyltrisiloxane | - | - | - | - | - | - | balance | - |
| Total | 100.0 | 100.0 | 100.0 | 100.0 | 100.0 | 100.0 | 100.0 | 100.0 |

**[Table 11]**

| Formulation (%) | Example | | | | | | Comparative Example | |
|---|---|---|---|---|---|---|---|---|
| | 27 | 28 | 29 | 30 | 31 | 32 | 25 | 26 |
| Synthesis Example 4 | 10 | 10 | 10 | 10 | 10 | 10 | - | - |
| Comparative Synthesis Example 2 | - | - | - | - | - | - | 10 | 10 |
| MT-100TV (*1) | - | - | - | - | - | - | 40 | - |
| MZ-306X (*2) | 50 | 50 | 50 | 50 | 50 | 50 | - | 50 |
| KF-56A (*3) | 12 | - | balance | - | - | - | 12 | 12 |
| Ethylhexyl methoxycinnamate | 8 | - | - | - | - | - | 8 | 8 |
| Dimethicone (2 cs) | balance | - | - | - | - | - | - | - |
| Dodecane | - | balance | - | - | - | - | - | - |
| Ethylhexyl palmitate | - | - | - | balance | - | - | balance | balance |
| Ethylmethicone | - | - | - | - | balance | - | - | - |
| Ethyltrisiloxane | - | - | - | - | - | balance | - | - |
| Total | 100.0 | 100.0 | 100.0 | 100.0 | 100.0 | 100.0 | 100.0 | 100.0 |

Table 10 and 11
(*1) Stearic acid (labeling name, INCI: Stearic Acid) and Al hydroxide (labeling name, INCI: Aluminum Hydroxide)-treated microparticulate titanium oxide (labeling name, INCI: Titanium Dioxide)
(*2) Triethoxycaprylylsilane (labeling name, INCI: Triethoxycaprylylsilane)-treated microparticulate zinc oxide (labeling name, INCI: Zinc Oxide)
(*3) Diphenylsiloxy phenyl trimethicone (labeling name, INCI: Diphenylsiloxy phenyl trimethicone)

Examples 17 to 30 all yielded low viscosity emulsions which had satisfactory stability with time.

### [Example 33]

A sun care cream was prepared according to the formulation in Table 12.

**[Table 12]**

| Formulation (%) | | Example 33 |
|---|---|---|
| 1 | Crosslinked polyether-modified silicone mixture (*1) | 2.0 |
| 2 | Crosslinked phenyl-modified silicone mixture (*2) | 2.0 |
| 3 | Alkyl/polyether-co-modified silicone (*3) | 1.5 |
| 4 | Decamethylcyclopentasiloxane | 23.5 |
| 5 | (C13-15) alkanes | 3.0 |
| 6 | Disteardimonium hectorite | 0.8 |
| 7 | Ethylhexyl methoxycinnamate | 3.0 |
| 8 | Ethylhexyl salicylate | 2.0 |
| 9 | Bis-ethylhexyloxyphenol methoxyphenyl triazine | 2.0 |
| 10 | Diphenylsiloxy phenyl trimethicone (*4) | 5.0 |
| 11 | Phenyl-modified spherical silicone composite powder (*5) | 0.5 |
| 12 | Dispersion of Example 19 | 12.0 |
| 13 | 1,3-butylene glycol | 3.0 |
| 14 | Ethanol | 5.0 |
| 15 | Sodium citrate | 0.2 |
| 16 | NaCl | 0.5 |
| 17 | Purified water | 34.0 |
| Total | | 100.0 |

| | | |
|---|---|---|
| (*1) KSG-240 from Shin-Etsu Chemical Co., Ltd. (mixture of decamethylpentasiloxane 75-85% and crosslinked polyether-modified silicone 15-25%) (*2) KSG-18A from Shin-Etsu Chemical Co., Ltd. (mixture of diphenylsiloxy phenyl trimethicone 80-90% and crosslinked phenyl-modified silicone 10-20%) (*3) KF-6048 from Shin-Etsu Chemical Co., Ltd. (*4) KF-56A from Shin-Etsu Chemical Co., Ltd. (*5) KSP-300 from Shin-Etsu Chemical Co., Ltd. | | |

### (Preparation method)

Components 1 to 11 were mixed until uniform. After uniform mixing, components 13 to 17 were slowly added to the mix and agitated to form an emulsion. Component 12 was added to the emulsion and mixed. A predetermined vessel was filled with the emulsion, obtaining a sun care cream. The resulting cream spread lightly, was dry and non-sticky, and presented a pleasant feeling on use.

### [Example 34]

A sun care cream was prepared according to the formulation in Table 13.

**[Table 13]**

| Formulation (%) | | Example 34 |
|---|---|---|
| 1 | Crosslinked polyether-modified silicone mixture (*1) | 2.0 |
| 2 | Crosslinked phenyl-modified silicone mixture (*2) | 2.0 |
| 3 | Alkyl/polyether-co-modified silicone (*3) | 1.5 |
| 4 | Decamethylcyclopentasiloxane | 38.0 |
| 5 | Dodecane | 3.0 |
| 6 | Disteardimonium hectorite | 0.8 |
| 7 | Ethylhexyl methoxycinnamate | 2.0 |
| 8 | Diethylaminohydroxybenzoyl hexyl benzoate | 0.5 |
| 9 | t-butylmethoxydibenzoylmethane | 0.5 |
| 10 | Diphenylsiloxyphenyl trimethicone (*4) | 5.0 |
| 11 | Phenyl-modified spherical silicone composite powder (*5) | 1.0 |
| 12 | Dispersion of Example 20 | 1.0 |
| 13 | 1,3-butylene glycol | 3.0 |
| 14 | Ethanol | 5.0 |
| 15 | Sodium citrate | 0.2 |
| 16 | NaCl | 0.5 |
| 17 | 2K glycyrrhizinate | 0.2 |
| 18 | Purified water | balance |
| Total | | 100.0 |
| Viscosity (mPa·s) | | 30.1 |

| | | |
|---|---|---|
| (*1) KSG-210 from Shin-Etsu Chemical Co., Ltd. (mixture of dimethylpolysiloxane 70-80% and crosslinked polyether-modified silicone 20-30%) (*2) KSG-16 from Shin-Etsu Chemical Co., Ltd. (mixture of dimethylpolysiloxane 70-80% and crosslinked dimethylpolysiloxane 20-30%) (*3) KF-6048 from Shin-Etsu Chemical Co., Ltd. (*4) KF-56A from Shin-Etsu Chemical Co., Ltd. (*5) KSP-300 from Shin-Etsu Chemical Co., Ltd. | | |

### (Preparation method)

Components 1 to 11 were mixed until uniform. After uniform mixing, components 13 to 18 were slowly added to the mix and agitated to form an emulsion. Component 12 was added to the emulsion and mixed. A predetermined vessel was filled with the emulsion, obtaining a sun care cream. The resulting cream spread lightly, was dry and non-sticky, and presented a pleasant feeling on use.

### [Example 35]

A sun care cream was prepared according to the formulation in Table 14.

**[Table 14]**

| Formulation (%) | | Example 35 |
|---|---|---|
| 1 | Crosslinked polyether-modified silicone mixture (*1) | 2.0 |
| 2 | Crosslinked dimethylpolysiloxane mixture (*2) | 2.0 |
| 3 | Alkyl/polyether-co-modified silicone (*3) | 1.5 |
| 4 | Dimethicone (6 cs) | 22.5 |
| 5 | Isohexadecane | 3.0 |
| 6 | Ethylhexyl methoxycinnamate | 1.0 |
| 7 | Polysilicone-15 | 0.5 |
| 7 | Homosalate | 0.5 |
| 8 | Diphenylsiloxyphenyl trimethicone (*4) | 5.0 |
| 9 | Spherical silicone composite powder (*5) | 2.0 |
| 10 | Dispersion of Example 21 | 7.5 |
| 11 | glycerin | 3.0 |
| 12 | Sodium hyaluronate | 0.2 |
| 13 | Ethanol | 5.0 |
| 14 | Sodium citrate | 0.2 |
| 15 | Magnesium sulfate | 0.5 |
| 16 | Purified water | 43.6 |
| Total | | 100.0 |

| | | |
|---|---|---|
| (*1) KSG-210 from Shin-Etsu Chemical Co., Ltd. (mixture of dimethylpolysiloxane 70-80% and crosslinked polyether-modified silicone 20-30%) (*2) KSG-16 from Shin-Etsu Chemical Co., Ltd. (mixture of dimethylpolysiloxane 70-80% and crosslinked dimethylpolysiloxane 20-30%) (*3) KF-6048 from Shin-Etsu Chemical Co., Ltd. (*4) KF-56A from Shin-Etsu Chemical Co., Ltd. (*5) KSP-300 from Shin-Etsu Chemical Co., Ltd. | | |

### (Preparation method)

Components 1 to 9 were mixed until uniform. After uniform mixing, components 11 to 16 were slowly added to the mix and agitated to form an emulsion. Component 10 was added to the emulsion and mixed. A predetermined vessel was filled with the emulsion, obtaining a sun care cream. The resulting cream spread lightly, was dry and non-sticky, and presented a pleasant feeling on use.

### [Example 36]

A sun care cream was prepared according to the formulation in Table 15.

**[Table 15]**

| Formulation (%) | | Example 36 |
|---|---|---|
| 1 | Crosslinked alkyl/polyglycerin-co-modified silicone mixture (*1) | 2.0 |
| 2 | Crosslinked alkyl-modified silicone mixture (*2) | 2.0 |
| 3 | Synthesis Example 5 | 4.0 |
| 4 | Neopentyl glycol diethylhexanoate | 8.0 |
| 5 | Isotridecyl isononanoate | 13.0 |
| 6 | Diphenylsiloxyphenyl trimethicone (*3) | 5.0 |
| 7 | Alkyl-modified spherical silicone composite powder (*4) | 2.0 |
| 8 | Polymethylsilsesquioxane (*5) | 0.5 |
| 9 | Dispersion of Example 26 | 12.0 |
| 10 | Ethylhexyl glycerin | 3.0 |
| 11 | Sorbitol | 0.2 |
| 12 | Ethanol | 5.0 |
| 13 | Sodium citrate | 0.2 |
| 14 | Sodium chloride | 0.5 |
| 15 | Hydroxyethyl cellulose | 0.3 |
| 16 | Arbutin | 0.5 |
| 17 | Allantoin | 0.1 |
| 18 | Phenoxyethanol | 0.3 |
| 19 | Methylparaben | 0.1 |
| 20 | Purified water | balance |
| Total | | 100.0 |

| | | |
|---|---|---|
| (*1) KSG-820 from Shin-Etsu Chemical Co., Ltd. (mixture of isododecane 70-80% and crosslinked alkyl/glycerin-co-modified silicone 20-30%) (*2) KSG-44 from Shin-Etsu Chemical Co., Ltd. (mixture of squalane 65-75% and crosslinked alkyl-modified dimethylpolysiloxane 25-35%) (*3) KF-56A from Shin-Etsu Chemical Co., Ltd. (*4) KSP-441 from Shin-Etsu Chemical Co., Ltd. (*5) KMP-591 from Shin-Etsu Chemical Co., Ltd. | | |

### (Preparation method)

Components 1 to 8 were mixed until uniform. After uniform mixing, components 10 to 20 were slowly added to the mix and agitated to form an emulsion. Component 9 was added to the emulsion and mixed. A predetermined vessel was filled with the emulsion, obtaining a sun care cream. The resulting cream spread lightly, was dry and non-sticky, and presented a pleasant feeling on use.

### [Example 37]

A sun care cream was prepared according to the formulation in Table 16.

**[Table 16]**

| Formulation (%) | | Example 37 |
|---|---|---|
| 1 | Crosslinked polyether-modified silicone mixture (*1) | 2.0 |
| 2 | Crosslinked phenyl-modified silicone mixture (*2) | 2.0 |
| 3 | Synthesis Example 5 | 3.0 |
| 4 | 2-ethylhexyl palmitate | 8.0 |
| 5 | Dimethicone | 13.0 |
| 6 | Isononyl isononanoate | 7.0 |
| 7 | Triethylhexanoin | 2.0 |
| 8 | Spherical silicone composite powder (*3) | 2.0 |
| 9 | Acrylate silicone graft copolymer solution (*4) | 0.5 |
| 10 | Dispersion of Example 24 | 12.0 |
| 11 | Dispersion of Example 32 | 12.0 |
| 12 | Ethanol | 5.0 |
| 13 | Sodium citrate | 0.2 |
| 14 | Sodium chloride | 0.5 |
| 15 | Purified water | balance |
| Total | | 100.0 |

| | | |
|---|---|---|
| (*1) KSG-210 from Shin-Etsu Chemical Co., Ltd. (mixture of dimethylpolysiloxane 70-80% and crosslinked polyether-modified silicone 20-30%) (*2) KSG-18A from Shin-Etsu Chemical Co., Ltd. (mixture of diphenylsiloxyphenyl trimethicone 80-90% and crosslinked phenyl-modified silicone 10-20%) (*3) KSP-101 from Shin-Etsu Chemical Co., Ltd. (*4) KP-545L from Shin-Etsu Chemical Co., Ltd. (solution of dimethicone 60% and acrylate silicone graft copolymer 40%) | | |

### (Preparation method)

Components 1 to 9 were mixed until uniform. After uniform mixing, components 12 to 15 were slowly added to the mix and agitated to form an emulsion. Components 10 and 11 was added to the emulsion and mixed. A predetermined vessel was filled with the emulsion, obtaining a sun care cream. The resulting cream spread lightly, was dry and non-sticky, and presented a pleasant feeling on use.

### [Example 38]

A sun care emulsion was prepared according to the formulation in Table 17.

**[Table 17]**

| Formulation (%) | | Example 38 |
|---|---|---|
| 1 | Crosslinked dimethylpolysiloxane mixture (*1) | 6.0 |
| 2 | Synthesis Example 5 | 1.5 |
| 3 | Decamethylcyclopentasiloxane | 5.0 |
| 4 | Dimethicone (6 cs) | 3.0 |
| 5 | Isotridecyl isononanoate | 4.8 |
| 6 | Spherical silicone composite powder (*2) | 1.0 |
| 7 | Dispersion of Example 20 | 25.0 |
| 8 | Dispersion of Example 27 | 25.0 |
| 9 | 1,3-butylene glycol | 2.0 |
| 10 | Ethanol | 2.0 |
| 11 | Sodium citrate | 0.2 |
| 12 | Sodium chloride | 0.5 |
| 13 | Purified water | 24.0 |
| Total | | 100.0 |

| | | |
|---|---|---|
| (*1) KSG-16 from Shin-Etsu Chemical Co., Ltd. (mixture of dimethylpolysiloxane 70-80% and crosslinked dimethylpolysiloxane 20-30%) (*2) KSP-102 from Shin-Etsu Chemical Co., Ltd. | | |

### (Preparation method)

Components 1 to 6 were thoroughly mixed until uniform. After uniform mixing, components 9 to 13 were slowly added to the mix and agitated to form an emulsion. Components 7 and 8 were added to the emulsion and mixed. A predetermined vessel was filled with the emulsion and stainless steel balls, obtaining a sun care emulsion. The resulting emulsion spread lightly, was dry and non-sticky, and presented a pleasant feeling on use.

### [Example 39]

A foundation was prepared according to the formulation in Table 18.

**[Table 18]**

| Formulation (%) | | Example 39 |
|---|---|---|
| 1 | Crosslinked polyether-modified silicone mixture (*1) | 3.0 |
| 2 | Crosslinked dimethylpolysiloxane mixture (*2) | 5.0 |
| 3 | Synthesis Example 5 | 3.0 |
| 4 | Dimethicone | 13.8 |
| 5 | Decamethylcyclopentasiloxane | 7.5 |
| 6 | Neopentylglycol diethylhexanoate | 6.0 |
| 7 | Synthesis Example 4 | 0.3 |
| 8 | Silicone-treated titanium oxide (*3) | 8.5 |
| 9 | Silicone-treated red iron oxide (*3) | 0.5 |
| 10 | Silicone-treated yellow iron oxide (*3) | 0.9 |
| 11 | Silicone-treated black iron oxide (*3) | 0.1 |
| 12 | Isotridecyl isononanoate | 1.7 |
| 13 | 1,3-butylene glycol | 5.0 |
| 14 | Sodium citrate | 0.2 |
| 15 | Sodium chloride | 1.0 |
| 16 | Purified water | 43.5 |
| Total | | 100.0 |

| | | |
|---|---|---|
| (*1) KSG-240 from Shin-Etsu Chemical Co., Ltd. (mixture of decamethylcyclopentasiloxane 75-85% and crosslinked polyether-modified silicone 15-25%) (*2) KSG-15 from Shin-Etsu Chemical Co., Ltd. (mixture of decamethylcyclopentasiloxane 90-96% and crosslinked dimethylpolysiloxane 4-10%) (*3) KTP-09W, R, Y and B (KF-9909-treated coloring inorganic pigments, W: white, R: red, Y: yellow, B: black) from Shin-Etsu Chemical Co., Ltd. | | |

### (Preparation method)

The foundation was obtained by
A: uniformly mixing components 1 to 6,
B: uniformly mixing components 13 to 16,
C: uniformly mixing components 7 to 12 and roll milling the mix,
D: slowly adding B to A, emulsifying and then adding C.

The resulting foundation spread lightly, was dry and non-sticky, and showed satisfactory pigment dispersion.

### [Example 40]

A foundation was prepared according to the formulation in Table 19.

**[Table 19]**

| Formulation (%) | | Example 40 |
|---|---|---|
| 1 | Crosslinked polyether-modified silicone mixture (*1) | 3.0 |
| 2 | Crosslinked phenyl-modified silicone mixture (*2) | 3.0 |
| 3 | Synthesis Example 4 | 5.0 |
| 4 | Dimethicone | 5.0 |
| 5 | Ethylhexyl methoxycinnamate | 2.0 |
| 6 | Ethylhexyl salicylate | 2.0 |
| 7 | Octocrylene | 2.0 |
| 8 | Diphenylsiloxyphenyl trimethicone | 6.0 |
| 9 | Synthesis Example 4 | 10.0 |
| 10 | Methylhydrogenpolysiloxane-treated titanium oxide (*3) | 17.0 |
| 11 | Methylhydrogenpolysiloxane-treated red iron oxide (*3) | 1.0 |
| 12 | Methylhydrogenpolysiloxane-treated yellow iron oxide (*3) | 1.8 |
| 13 | Methylhydrogenpolysiloxane-treated black iron oxide (*3) | 0.2 |
| 14 | Glyceryl tri(2-ethylhexanoate) | 5.0 |
| 15 | 1,3-butylene glycol | 2.0 |
| 16 | Sodium citrate | 0.2 |
| 17 | Sodium chloride | 1.0 |
| 18 | Purified water | 33.8 |
| Total | | 100.0 |

| | | |
|---|---|---|
| (*1) KSG-210 from Shin-Etsu Chemical Co., Ltd. (mixture of dimethylpolysiloxane 70-80% and crosslinked polyether-modified silicone 20-30%) (*2) KSG-18A from Shin-Etsu Chemical Co., Ltd. (mixture of diphenylsiloxyphenyl trimethicone 80-90% and crosslinked phenyl-modified silicone 10-20%) (*3) treated with KF-99 from Shin-Etsu Chemical Co., Ltd. | | |

### (Preparation method)

The foundation was obtained by
A: uniformly mixing components 1 to 8,
B: uniformly mixing components 15 to 18,
C: uniformly mixing components 9 to 14 and roll milling the mix,
D: slowly adding B to A, emulsifying and then adding C.

The resulting foundation spread lightly, was dry and non-sticky, and showed satisfactory pigment dispersion.

### [Example 41]

A foundation was prepared according to the formulation in Table 20.

**[Table 20]**

| Formulation (%) | | Example 41 |
|---|---|---|
| 1 | Crosslinked polyether-modified silicone mixture (*1) | 3.0 |
| 2 | Crosslinked dimethylpolysiloxane mixture (*2) | 5.0 |
| 3 | Alkyl/polyether-co-modified silicone (*3) | 3.0 |
| 4 | Dimethicone | 13.8 |
| 5 | Ethylhexyl methoxycinnamate | 5.0 |
| 6 | Diethylaminohydroxybenzoyl benzoate | 2.0 |
| 7 | Synthesis Example 4 | 1.5 |
| 8 | Silicone-treated titanium oxide (*4) | 8.5 |
| 9 | Silicone-treated red iron oxide (*4) | 0.5 |
| 10 | Silicone-treated yellow iron oxide (*4) | 0.9 |
| 11 | Silicone-treated black iron oxide (*4) | 0.1 |
| 12 | Silicone-treated microparticulate zinc oxide | 10.0 |
| 13 | Isononyl isononanoate | 4.0 |
| 14 | Diphenylsiloxyphenyl trimethicone (*5) | 6.0 |
| 15 | Sorbitol | 0.2 |
| 16 | Glycerin | 4.0 |
| 17 | Sodium citrate | 0.2 |
| 18 | Sodium chloride | 1.0 |
| 19 | Purified water | 31.3 |
| Total | | 100.0 |

| | | |
|---|---|---|
| (*1) KSG-210 from Shin-Etsu Chemical Co., Ltd. (mixture of dimethylpolysiloxane 70-80% and crosslinked polyether-modified silicone 20-30%) (*2) KSG-16 from Shin-Etsu Chemical Co., Ltd. (mixture of dimethylpolysiloxane 70-80% and crosslinked dimethylpolysiloxane 20-30%) (*3) KF-6048 from Shin-Etsu Chemical Co., Ltd. (*4) KTP-09W, R, Y and B (KF-9909-treated coloring inorganic pigments, W: white, R: red, Y: yellow, B: black) from Shin-Etsu Chemical Co., Ltd. (*5) KF-56A from Shin-Etsu Chemical Co., Ltd. | | |

### (Preparation method)

The foundation was obtained by
A: uniformly mixing components 1 to 6,
B: uniformly mixing components 15 to 19,
C: uniformly mixing components 7 to 14 and roll milling the mix,
D: slowly adding B to A, emulsifying and then adding C.

The resulting foundation spread lightly, was dry and non-sticky, and showed satisfactory pigment dispersion.

### [Example 42]

A foundation was prepared according to the formulation in Table 21.

**[Table 21]**

| Formulation (%) | | Example 42 |
|---|---|---|
| 1 | Crosslinked poly glycerin-modified silicone mixture (*1) | 4.0 |
| 2 | Crosslinked phenyl-modified silicone mixture (*2) | 5.0 |
| 3 | Synthesis Example 5 | 3.0 |
| 4 | Dispersion of Example 21 | 12.0 |
| 5 | Ethylhexyl methoxycinnamate | 7.5 |
| 6 | Diphenylsiloxyphenyl trimethicone | 6.0 |
| 7 | Synthesis Example 4 | 2.0 |
| 8 | Acrylate silicone-treated titanium oxide (*3) | 12.8 |
| 9 | Acrylate silicone-treated red iron oxide (*3) | 0.8 |
| 10 | Acrylate silicone-treated yellow iron oxide (*3) | 1.4 |
| 11 | Acrylate silicone-treated black iron oxide (*3) | 0.2 |
| 12 | Neopentylglycol dioctanoate | 1.7 |
| 13 | 1,3-butylene glycol | 5.0 |
| 14 | Sodium citrate | 0.2 |
| 15 | Sodium chloride | 1.0 |
| 16 | Purified water | 37.6 |
| Total | | 100.0 |

| | | |
|---|---|---|
| (*1) KSG-710 from Shin-Etsu Chemical Co., Ltd. (mixture of dimethylpolysiloxane 70-80% and crosslinked polyglycerin-modified silicone 20-30%) (*2) KSG-18A from Shin-Etsu Chemical Co., Ltd. (mixture of diphenylsiloxyphenyl trimethicone 80-90% and crosslinked phenyl-modified silicone 10-20%) (*3) treated with KP-574 from Shin-Etsu Chemical Co., Ltd. | | |

### (Preparation method)

The foundation was obtained by
A: uniformly mixing components 1 to 6,
B: uniformly mixing components 13 to 16,
C: uniformly mixing components 7 to 12 and roll milling the mix,
D: slowly adding B to A, emulsifying and then adding C.

The resulting foundation spread lightly, was dry and non-sticky, and showed satisfactory pigment dispersion.

### [Example 43]

A foundation was prepared according to the formulation in Table 22.

**[Table 22]**

| Formulation (%) | | Example 43 |
|---|---|---|
| 1 | Crosslinked alkyl/polyglycerin-co-modified silicone mixture (*1) | 3.0 |
| 2 | Crosslinked alkyl-modified silicone mixture (*2) | 5.0 |
| 3 | Silicone branched alkyl/polyglycerin-co-modified silicone (*3) | 3.0 |
| 4 | Cetyl 2-ethylhexanoate | 3.0 |
| 5 | Dispersion of Example 17 | 20.0 |
| 6 | Dispersion of Example 24 | 15.0 |
| 7 | Synthesis Example 5 | 0.3 |
| 8 | Silicone-treated titanium oxide (*4) | 8.5 |
| 9 | Silicone-treated red iron oxide (*4) | 0.5 |
| 10 | Silicone-treated yellow iron oxide (*4) | 0.9 |
| 11 | Silicone-treated black iron oxide (*4) | 0.1 |
| 12 | Isotridecyl isononanoate | 1.7 |
| 13 | 1,3-butylene glycol | 5.0 |
| 14 | Sodium citrate | 0.2 |
| 15 | Sodium chloride | 1.0 |
| 16 | Purified water | 32.8 |
| Total | | 100.0 |

| | | |
|---|---|---|
| (*1) KSG-840 from Shin-Etsu Chemical Co., Ltd. (mixture of squalane 65-75% and crosslinked alkyl/polyglycerin-co-modified silicone 25-35%) (*2) KSG-44 from Shin-Etsu Chemical Co., Ltd. (mixture of squalane 65-75% and crosslinked alkyl-modified silicone 25-35%) (*3) KF-6105 from Shin-Etsu Chemical Co., Ltd. (*4) KTP-09W, R, Y and B (KF-9909-treated coloring inorganic pigments, W: white, R: red, Y: yellow, B: black) from Shin-Etsu Chemical Co., Ltd. | | |

### (Preparation method)

The foundation was obtained by
A: uniformly mixing components 1 to 4,
B: uniformly mixing components 13 to 16,
C: uniformly mixing components 7 to 12 and roll milling the mix,
D: slowly adding B to A, emulsifying, adding C and components 5 and 6, and uniformly mixing.

The resulting foundation spread lightly, was dry and non-sticky, and showed satisfactory pigment dispersion.

### [Example 44]

A foundation was prepared according to the formulation in Table 23.

**[Table 23]**

| Formulation (%) | | Example 44 |
|---|---|---|
| 1 | Crosslinked polyether-modified silicone mixture (*1) | 3.0 |
| 2 | Crosslinked dimethylpolysiloxane mixture (*2) | 5.0 |
| 3 | Disteardimonium hectorite | 1.2 |
| 4 | Decamethylcyclopentasiloxane | 12.3 |
| 5 | Ethylhexyl methoxycinnamate | 7.5 |
| 6 | Synthesis Example 4 | 5.0 |
| 7 | Synthesis Example 4 | 2.0 |
| 8 | Synthesis Example 2 | 3.0 |
| 9 | Trimethoxysilyl dimethicone-treated titanium oxide | 5.1 |
| 10 | Trimethoxysilyl dimethicone-treated red iron oxide | 0.3 |
| 11 | Trimethoxysilyl dimethicone-treated yellow iron oxide | 0.5 |
| 12 | Trimethoxysilyl dimethicone-treated black iron oxide | 0.1 |
| 13 | Metal soap-treated microparticulate titanium oxide | 10.0 |
| 14 | Isotridecyl isononanoate | 4.0 |
| 15 | Diphenylsiloxyphenyl trimethicone (*3) | 6.0 |
| 16 | Phenoxyethanol | 0.2 |
| 17 | DPG | 4.0 |
| 18 | Sodium citrate | 0.2 |
| 19 | Magnesium sulfate | 1.0 |
| 20 | Water | 29.6 |
| Total | | 100.0 |

| | | |
|---|---|---|
| (*1) KSG-240 from Shin-Etsu Chemical Co., Ltd. (mixture of decamethylcyclopentasiloxane 75-85% and crosslinked polyether-modified silicone 15-25%) (*2) KSG-15 from Shin-Etsu Chemical Co., Ltd. (mixture of decamethylcyclopentasiloxane 90-96% and crosslinked dimethylpolysiloxane 4-10%) (*3) KF-56A from Shin-Etsu Chemical Co., Ltd. | | |

### (Preparation method)

The foundation was obtained by
A: uniformly mixing components 1 to 6,
B: uniformly mixing components 13 to 20,
C: uniformly mixing components 7 to 13 and roll milling the mix,
D: slowly adding B to A, emulsifying, and then adding C.

The resulting foundation spread lightly, was dry and non-sticky, and showed satisfactory pigment dispersion.

### [Example 45]

A sun care cream was prepared according to the formulation in Table 24.

**[Table 24]**

| Formulation (%) | | Example 45 |
|---|---|---|
| 1 | Dispersion of Example 25 | 10.0 |
| 2 | Dispersion of Example 31 | 10.0 |
| 3 | Dimethicone (6 cs) | 2.0 |
| 4 | Diphenylsiloxyphenyl trimethicone (*1) | 0.5 |
| 5 | Ethylhexyl methoxycinnamate | 0.5 |
| 6 | (Ammonium acryloyldimethyl taurate/VP) copolymer | 2.0 |
| 7 | (Acrylamide/sodium acryloyldimethyl taurate) copolymer | 0.4 |
| 8 | Polyglyceryl-10 myristate | 4.0 |
| 9 | Polyglyceryl-10 dimyristate | 3.0 |
| 10 | 1,3-butylene glycol | 0.2 |
| 11 | Ethanol | 4.0 |
| 12 | Citric acid | 0.0 |
| 13 | Phenoxyethanol | 0.2 |
| 14 | Purified water | balance |
| Total | | 100.0 |

| | | |
|---|---|---|
| (*1) KSG-56A from Shin-Etsu Chemical Co., Ltd. | | |

### (Preparation method)

The sun care cream was obtained by
A: uniformly mixing components 6 to 14,
B: uniformly mixing components 1 to 5,
C: slowly adding B to A and emulsifying.

The resulting cream spread lightly, was dry and non-sticky, and showed satisfactory pigment dispersion.

### [Example 46]

A foundation was prepared according to the formulation in Table 25.

**[Table 25]**

| Formulation (%) | | Example 46 |
|---|---|---|
| 1 | Crosslinked poly glycerin-modified silicone mixture (*1) | 3.0 |
| 2 | Crosslinked dimethylpolysiloxane mixture (*2) | 5.0 |
| 3 | Silicone branched alkyl/polyglycerin-co-modified silicone (*3) | 3.0 |
| 4 | Dimethicone (2 cs) | 6.8 |
| 5 | Acrylate silicone solution (*4) | 2.0 |
| 6 | Spherical silicone composite powder (*5) | 1.0 |
| 7 | Ethylhexyl methoxycinnamate | 5.0 |
| 8 | Silicone branched alkyl/polyether-co-modified silicone (*6) | 3.0 |
| 9 | Synthesis Example 4 | 1.0 |
| 10 | Silicone branched alkyl/polyglycerin-co-modified silicone (*3) | 0.5 |
| 11 | Caprylylsilane-treated titanium oxide (*7) | 8.5 |
| 12 | Caprylylsilane-treated red iron oxide (*7) | 0.5 |
| 13 | Caprylylsilane-treated yellow iron oxide (*7) | 0.9 |
| 14 | Caprylylsilane-treated black iron oxide (*7) | 0.1 |
| 15 | Caprylylsilane-treated microparticulate zinc oxide | 10.0 |
| 16 | Isotridecyl isononanoate | 4.0 |
| 17 | Diphenylsiloxyphenyl trimethicone (*8) | 6.0 |
| 18 | Sodium hyaluronate | 0.2 |
| 19 | Glycerin | 4.0 |
| 20 | Sodium citrate | 0.2 |
| 21 | Sodium chloride | 1.0 |
| 22 | Purified water | 34.3 |
| Total | | 100.0 |

| | | |
|---|---|---|
| (*1) KSG-710 from Shin-Etsu Chemical Co., Ltd. (mixture of dimethylpolysiloxane 70-80% and crosslinked polyglycerin-modified silicone 20-30%) (*2) KSG-15 from Shin-Etsu Chemical Co., Ltd. (mixture of decamethylcyclopentasiloxane 90-96% and crosslinked dimethylpolysiloxane 4-10%) (*3) KF-6105 from Shin-Etsu Chemical Co., Ltd. (*4) KP-545L from Shin-Etsu Chemical Co., Ltd. (solution of dimethicone (2 cs) 60% and acrylate silicone graft copolymer 40%) (*5) KSP-101 from Shin-Etsu Chemical Co., Ltd. (*6) KF-6038 from Shin-Etsu Chemical Co., Ltd. (*7) treated with AES-3083 from Shin-Etsu Chemical Co., Ltd. (*8) KF-56A from Shin-Etsu Chemical Co., Ltd. | | |

### (Preparation method)

The foundation was obtained by
A: uniformly mixing components 1 to 8,
B: uniformly mixing components 18 to 22,
C: uniformly mixing components 9 to 17 and roll milling the mix,
D: slowly adding B to A, emulsifying, and then adding C.

The resulting foundation spread lightly, was dry and non-sticky, and showed satisfactory pigment dispersion.

### [Example 47]

A foundation was prepared according to the formulation in Table 26.

**[Table 26]**

| Formulation (%) | | Example 47 |
|---|---|---|
| 1 | Crosslinked alkyl/polyether-co-modified silicone mixture (*1) | 3.0 |
| 2 | Crosslinked alkyl-modified dimethylpolysiloxane mixture (*2) | 5.0 |
| 3 | Disteardimonium hectorite | 1.2 |
| 4 | Cetyl 2-ethylhexanoate | 13.3 |
| 5 | Isododecane | 3.0 |
| 6 | Spherical silicone composite powder (*3) | 1.0 |
| 7 | Phenyl-modified spherical silicone composite powder (*4) | 1.0 |
| 8 | Octocrylene | 5.0 |
| 9 | Silicone branched alkyl/polyether-modified silicone (*5) | 3.0 |
| 10 | Synthesis Example 4 | 1.5 |
| 11 | Caprylylsilane-treated titanium oxide (*6) | 8.5 |
| 12 | Caprylylsilane-treated red iron oxide (*6) | 0.5 |
| 13 | Caprylylsilane-treated yellow iron oxide (*6) | 0.9 |
| 14 | Caprylylsilane-treated black iron oxide (*6) | 0.1 |
| 15 | Caprylylsilane-treated microparticulate zinc oxide | 10.0 |
| 16 | Isotridecyl isononanoate | 10.0 |
| 17 | Ethylhexylglycerin | 1.2 |
| 18 | 1,3-butylene glycol | 3.0 |
| 19 | Sodium citrate | 0.2 |
| 20 | Sodium chloride | 1.0 |
| 21 | Purified water | 27.6 |
| Total | | 100.0 |
| Viscosity (Pa-s) | | 34.5 |

| | | |
|---|---|---|
| (*1) KSG-310 from Shin-Etsu Chemical Co., Ltd. (mixture of mineral oil 65-75% and crosslinked alkyl/polyether-co-modified silicone 25-35%) (*2) KSG-41A from Shin-Etsu Chemical Co., Ltd. (mixture of mineral oil 70-80% and crosslinked alkyl-modified dimethylpolysiloxane 20-30%) (*3) KSP-105 from Shin-Etsu Chemical Co., Ltd. (*4) KSP-300 from Shin-Etsu Chemical Co., Ltd. (*5) KF-6038 from Shin-Etsu Chemical Co., Ltd. (*6) treated with AES-3083 from Shin-Etsu Chemical Co., Ltd. | | |

### (Preparation method)

The foundation was obtained by
A: uniformly mixing components 1 to 9,
B: uniformly mixing components 17 to 21,
C: uniformly mixing components 10 to 16 and roll milling the mix,
D: slowly adding B to A, emulsifying, and then adding C.

The resulting foundation spread lightly, was dry and non-sticky, and showed satisfactory pigment dispersion.

### [Examples 48, 49 and Comparative Examples 27, 28]

A foundation was prepared by preparing a pigment paste according to the formulation in Table 27 and combining the paste according to the formulation in Table 28.

**[Table 27]**

| [Pigment paste formulation] | | | |
|---|---|---|---|
| Formulation (%) | | Example 48 | Comparative Example 27 |
| 1 | KTP-09W (*1) | 8.5 | 8.5 |
| 2 | KTP-09R (*2) | 0.5 | 0.5 |
| 3 | KTP-09Y (*2) | 0.9 | 0.9 |
| 4 | KTP-09B (*2) | 0.1 | 0.1 |
| 5 | MT-100Z (*3) | 10.0 | 10.0 |
| 6 | Synthesis Example 4 | 1.5 | - |
| 7 | Comparative Synthesis Example 2 | - | 1.5 |
| 8 | KF-56A (*4) | 6.0 | 6.0 |
| 9 | Isotridecyl Isononanoate | 4.0 | 4.0 |
| Total | | 31.5 | 31.5 |

| | | | |
|---|---|---|---|
| (*1) titanium oxide (labeling name, INCI: Titanium Dioxide) treated with triethoxysilylethyl polydimethylsiloxyethyl hexyl dimethicone (labeling name, INCI: Triethoxysilylethyl Polydimethylsiloxyethyl Hexyl Dimethicone) (*2) iron oxide (labeling name, INCI: Iron Oxides) treated with triethoxysilylethyl polydimethylsiloxyethyl hexyl dimethicone (labeling name, INCI: Triethoxysilylethyl Polydimethylsiloxyethyl Hexyl Dimethicone) (*3) microparticulate titanium oxide (labeling name, INCI: Titanium Dioxide) treated with stearic acid (labeling name, INCI: Stearic Acid) and Al hydroxide (labeling name, INCI: Aluminum Hydroxide) (*4) KF-56A from Shin-Etsu Chemical Co., Ltd. | | | |

### (Preparation method)

The pigment paste was prepared by
A: uniformly mixing components 1 to 9 and roll milling the mix.

**[Table 28]**

| [Foundation formulation] | | | |
|---|---|---|---|
| Formulation (%) | | Example 49 | Comparative Example 28 |
| 1 | KSG-210 (*1) | 3.0 | 3.0 |
| 2 | KSG-15 (*2) | 5.0 | 5.0 |
| 3 | Disteardimonium hectorite | 1.2 | 1.2 |
| 4 | Cyclopentasiloxane | 13.8 | 13.8 |
| 5 | Ethylhexyl methoxycinnamate | 7.5 | 7.5 |
| 6 | KF-6038 (*3) | 3.0 | 3.0 |
| 7 | Pigment paste of Example 48 | 31.5 | - |
| 8 | Pigment paste of Comparative Example 27 | - | 31.5 |
| 9 | Phenoxyethanol | 0.2 | 0.2 |
| 10 | DPG | 4.0 | 4.0 |
| 11 | Na citrate | 0.2 | 0.2 |
| 12 | Mg sulfate | 1.0 | 1.0 |
| 13 | Water | 29.6 | 29.6 |
| Total | | 100.0 | 100.0 |
| Evaluation of dispersion | | ⊚ | Δ |

| | | | |
|---|---|---|---|
| (*1) mixture of (dimethicone/PEG-10/15) crosspolymer (labeling name, INCI: Dimethicone/(PEG-10/15) crosspolymer) 25% and dimethicone (labeling name, INCI: Dimethicone) 75% (*2) mixture of cyclopentasiloxane (labeling name, INCI: Cyclopentasiloxane) 93% and (dimethicone/vinyl dimethicone) crosspolymer (labeling name, INCI: Dimethicone/Vinyl Dimethicone Crosspolymer) 7%) (*3) Lauryl PEG-9 polydimethylsiloxyethyl dimethicone (labeling name, INCI: Lauryl PEG-9 Polydimethylsiloxyethyl Dimethicone) | | | |

### (Preparation method)

The foundation was obtained by
A: uniformly mixing components 1 to 6,
B: uniformly mixing components 9 to 13,
C: slowly adding B to A, emulsifying, and then adding component 7 or 8.

### [Evaluation of dispersion]

The cosmetic composition thus obtained was measured for viscosity at 25°C after 1 week from preparation. The evaluation criterion is described below. The viscosity of a pigment paste was measured by using a Brookfield viscometer (DV-111 Ultra from Brookfield Engineering Laboratories Inc., with spindle T-F, and moving down the spindle at 2.2 cm per minute. The viscosity of a foundation was measured at 25°C using a B type viscometer (Model TVB-10, Toki Sangyo Co., Ltd.) according to JIS K 7117-1: 1999.

### [Pigment paste]

⊚: from 500 Pa·s to less than 600 Pa·s
○: from 600 Pa·s to less than 700 Pa·s
△: from 700 Pa·s to less than 800 Pa·s
×: 800 Pa·s or more

### [Foundation]

⊚: from 30,000 mPa·s to less than 40,000 mPa·s
○: from 40,000 mPa·s to less than 50,000 mPa·s
△: from 50,000 mPa·s to less than 60,000 mPa·s
×: 60,000 mPa·s or more

**[Table 29]**

| | Pigment paste | | Foundation | |
|---|---|---|---|---|
| | Example 48 | Comparative Example 25 | Example 49 | Comparative Example 26 |
| Evaluation of dispersion | ⊚ | × | ⊚ | Δ |

As is evident from Table 29, the pigment paste of Example 48 and the foundation of Example 49 using the same show better pigment dispersion than Comparative Examples 25 and 26.

### [Example 50]

A solid foundation was prepared according to the formulation in Table 30.

**[Table 30]**

| Formulation (%) | | Example 50 |
|---|---|---|
| 1 | Crosslinked alkyl-modified silicone mixture (*1) | 5.0 |
| 2 | Crosslinked silicone branched alkyl/polyether-co-modified silicone mixture (*2) | 2.5 |
| 3 | Synthetic wax | 6.0 |
| 4 | Inulin stearate | 2.0 |
| 5 | Glyceryl tri(caprylate/caprate) | 7.0 |
| 6 | Dicaprylyl carbonate | 7.0 |
| 7 | Spherical silicone composite powder (*3) | 0.5 |
| 8 | Long-chain alkyl-containing acrylic resin (*4) | 3.0 |
| 9 | Trimethylsiloxysilicic acid solution (*5) | 0.5 |
| 10 | Ethylhexyl methoxycinnamate | 3.0 |
| 11 | Neopentyl glycol diethylhexanoate | 5.0 |
| 12 | Isotridecyl isononanoate | 2.0 |
| 13 | Triethylhexanoin | 2.0 |
| 14 | Synthesis Example 4 | 0.2 |
| 15 | Silicone-treated titanium oxide (*6) | 8.5 |
| 16 | Silicone-treated red iron oxide (*6) | 0.5 |
| 17 | Silicone-treated yellow iron oxide (*6) | 0.9 |
| 18 | Silicone-treated black iron oxide (*6) | 0.1 |
| 19 | Silicone-treated microparticulate zinc oxide | 5.0 |
| 20 | Metal soap-treated microparticulate titanium oxide | 5.0 |
| 21 | DPG | 3.0 |
| 22 | 1,3-butylene glycol | 3.0 |
| 23 | Sodium citrate | 0.2 |
| 24 | Sodium chloride | 1.0 |
| 25 | Purified water | 27.1 |
| Total | | 100.0 |
| Viscosity (Pa-s) | | 34.50 |

| | | |
|---|---|---|
| (*1) KSG-44 from Shin-Etsu Chemical Co., Ltd. (mixture of squalane 65-75% and crosslinked alkyl-modified dimethylpolysiloxane 25-35%) (*2) KSG-320Z from Shin-Etsu Chemical Co., Ltd. (mixture of isododecane 70-80% and crosslinked silicone-branched alkyl/polyether-co-modified dimethylpolysiloxane 20-30%) (*3) KP-100 from Shin-Etsu Chemical Co., Ltd. (*4) KP-561P from Shin-Etsu Chemical Co., Ltd. (*5) X-21-5595 from Shin-Etsu Chemical Co., Ltd. (mixture of idododecane 40% and trimethylsiloxysilicic acid 60%) (*6) KTP-09W, R, Y and B (KF-9909-treated coloring inorganic pigments, W: white, R: red, Y: yellow, B: black) from Shin-Etsu Chemical Co., Ltd. | | |

### (Preparation method)

The solid foundation was obtained by
A: uniformly mixing components 1 to 9 at 90°C,
B: uniformly mixing components 21 to 25 at 85°C,
C: uniformly mixing components 10 to 20 and treating the mix on a homomixer,
D: slowly adding B to A at 85°C, emulsifying, and then adding C.

The resulting foundation spread lightly, was dry and non-sticky, and showed satisfactory pigment dispersion.

### [Example 51]

A cosmetic base was prepared according to the formulation in Table 31.

**[Table 31]**

| Formulation (%) | | Example 51 |
|---|---|---|
| 1 | Crosslinked silicone branched alkyl/polyglycerin-co-modified silicone mixture (*1) | 5.0 |
| 2 | Crosslinked silicone branched alkyl-modified silicone mixture (*2) | 3.0 |
| 3 | Alkyl/polyether-co-modified silicone (*3) | 1.5 |
| 4 | Dimethicone | 5.0 |
| 5 | 2-ethylhexyl palmitate | 8.0 |
| 6 | Ethylhexyl methoxycinnamate | 5.0 |
| 7 | KF-56A (*4) | 5.0 |
| 8 | Phenyl-modified spherical silicone composite powder (*5) | 2.0 |
| 9 | Acrylate silicone solution (*6) | 0.5 |
| 10 | Dispersion of Example 19 | 12.0 |
| 11 | Pentylene glycol | 3.0 |
| 12 | Ethanol | 5.0 |
| 13 | Sodium citrate | 0.2 |
| 14 | Sodium chloride | 0.5 |
| 15 | Hydroxyethyl cellulose | 0.3 |
| 16 | Methylparaben | 0.1 |
| 17 | Purified water | 43.9 |
| Total | | 100.0 |

| | | |
|---|---|---|
| (*1) KSG-850Z from Shin-Etsu Chemical Co., Ltd. (mixture of decamethylcyclopentasiloxane 70-80% and polyglycerin-modified silicone having silicone chains and alkyl chains branching from crosslinked silicone backbone 20-30%) (*2) KSG-045Z from Shin-Etsu Chemical Co., Ltd. (mixture of decamethylcyclopentasiloxane 75-85% and polyglycerin-modified silicone having silicone chains and alkyl chains branching from crosslinked silicone backbone 15-25%) (*3) KF-6048 from Shin-Etsu Chemical Co., Ltd. (*4) KF-56A from Shin-Etsu Chemical Co., Ltd. (*5) KSP-300 from Shin-Etsu Chemical Co., Ltd. (*6) KP-550 from Shin-Etsu Chemical Co., Ltd. (solution of isododecane 60% and acrylate silicone graft copolymer 40%) | | |

### (Preparation method)

The cosmetic base was obtained by
A: uniformly mixing components 1 to 9,
B: uniformly mixing components 11 to 17,
C: slowly adding B to A, emulsifying, and adding component 10.

The resulting cosmetic base spread lightly, was dry and non-sticky, and showed satisfactory pigment dispersion.

### [Example 52]

A non-aqueous concealer was prepared according to the formulation in Table 32.

**[Table 32]**

| Formulation (%) | | Example 52 |
|---|---|---|
| 1 | Spherical silicone composite powder (*1) | 25.0 |
| 2 | Phenyl-modified spherical silicone composite powder (*2) | 5.0 |
| 3 | Crosslinked dimethylpolysiloxane mixture (*3) | 5.0 |
| 4 | Dimethicone | 40.0 |
| 5 | Decamethylcyclopentasiloxane | 9.7 |
| 6 | Methylphenylpolysiloxane (*4) | 5.0 |
| 7 | Triethylhexanoin | 8.0 |
| 8 | Synthesis Example 4 | 2.0 |
| 9 | Silicone-treated titanium oxide (*5) | 0.3 |
| 10 | Silicone-treated red iron oxide (*5) | proper |
| 11 | Silicone-treated yellow iron oxide (*5) | proper |
| 12 | Silicone-treated black iron oxide (*5) | proper |
| Total | | 100.0 |

| | | |
|---|---|---|
| (*1) KSP-101 from Shin-Etsu Chemical Co., Ltd. (*2) KSP-300 from Shin-Etsu Chemical Co., Ltd. (*3) KSG-16 from Shin-Etsu Chemical Co., Ltd. (mixture of dimethylpolysiloxane 70-80% and crosslinked dimethylpolysiloxane 20-30%) (*4) KF-54HV from Shin-Etsu Chemical Co., Ltd. (*5) KTP-09W, R, Y and B (KF-9909-treated coloring inorganic pigments, W: white, R: red, Y: yellow, B: black) from Shin-Etsu Chemical Co., Ltd. | | |

### (Preparation method)

The non-aqueous concealer was obtained by
A: uniformly mixing components 1 to 7,
B: uniformly mixing components 8 to 12 and roll milling the mix,
Uniformly mixing A with B.

The resulting non-aqueous concealer spread lightly, was dry and non-sticky, and showed satisfactory pigment dispersion.

### [Example 53]

A lip stick was prepared according to the formulation in Table 33.

**[Table 33]**

| Formulation (%) | | Example 53 |
|---|---|---|
| 1 | Candelilla wax | 5.0 |
| 2 | Polyethylene | 5.0 |
| 3 | Microcrystalline wax | 6.0 |
| 4 | Long-chain alkyl-containing acrylic resin (*1) | 15.0 |
| 5 | Macadamia nut oil | 5.0 |
| 6 | Hydrogenated polyisobutene | 20.0 |
| 7 | Neopentyl glycol dicaprylate | 5.0 |
| 8 | Ethylhexyl methoxycinnamate | 4.0 |
| 9 | Neopentyl glycol diethylhexanoate | 2.0 |
| 10 | Isononyl isononanoate | 14.1 |
| 11 | Silicone-modified pullulan solution (*2) | 1.0 |
| 12 | Synthesis Example 5 | 3.0 |
| 13 | Diisostearyl malate | 6.0 |
| 14 | Mica | 1.0 |
| 15 | Red #201 | 0.5 |
| 16 | Red #202 | 0.5 |
| 17 | Yellow #4 | 1.6 |
| 18 | Silicone-treated titanium oxide (*3) | 4.0 |
| 19 | Silicone-treated red iron oxide (*3) | 1.0 |
| 20 | Silicone-treated black iron oxide (*3) | 0.3 |
| Total | | 100.0 |

| | | |
|---|---|---|
| (*1) KP-561P from Shin-Etsu Chemical Co., Ltd. (*2) TSPL-30-ID from Shin-Etsu Chemical Co., Ltd. (mixture of isododecane 70% and silicone-modified pullulan 30%) (*3) KTP-09W, R, Y and B (KF-9909-treated coloring inorganic pigments, W: white, R: red, Y: yellow, B: black) from Shin-Etsu Chemical Co., Ltd. | | |

### (Preparation method)

The lip stick was obtained by
A: uniformly mixing components 1 to 11 at 95°C,
B: uniformly mixing components 12 to 20 and roll milling the mix,
C: adding B to A at 70°C, mixing, and then adding C.

The resulting lip stick spread lightly, was dry and non-sticky, and showed satisfactory pigment dispersion.

### [Example 54]

A W/O mascara was prepared according to the formulation in Table 34.

**[Table 34]**

| Formulation (%) | | Example 54 |
|---|---|---|
| 1 | Isododecane | balance |
| 2 | Disteardimonium hectorite | 1.5 |
| 3 | Dextrin palmitate | 6.0 |
| 4 | Ceresin | 3.0 |
| 5 | Microcrystalline wax | 8.0 |
| 6 | Propylene carbonate | 0.5 |
| 7 | Trimethylsiloxycinnamate solution (*1) | 10.0 |
| 8 | Acrylate silicone solution (*2) | 10.0 |
| 9 | Isododecane | 10.0 |
| 10 | Synthesis Example 4 | 0.2 |
| 11 | Silicone-treated black iron oxide (*3) | 5.0 |
| 12 | Silicone-treated titanium oxide (*4) | 5.0 |
| 13 | Silicone branched alkyl/polyether-modified silicone (*5) | 0.2 |
| 14 | 1,3-butylene glycol | 2.0 |
| 15 | Sodium citrate | 0.1 |
| 16 | Sodium chloride | 0.1 |
| 17 | Purified water | 8.0 |
| Total | | 100.0 |

| | | |
|---|---|---|
| (*1) X-21-5595 from Shin-Etsu Chemical Co., Ltd. (mixture of isododecane 40% and trimethylsiloxysilicic acid 60%) (*2) KP-550 from Shin-Etsu Chemical Co., Ltd. (solution of idododecane 60% and acrylate silicone graft copolymer 40%) (*3) KTP-09B (KF-9909-treated coloring inorganic pigment) from Shin-Etsu Chemical Co., Ltd. (*4) treated with KF-9909 from Shin-Etsu Chemical Co., Ltd. (*5) KF-6038 from Shin-Etsu Chemical Co., Ltd. | | |

### (Preparation method)

The W/O mascara was obtained by
A: uniformly mixing components 1 to 8 at 90°C,
B: uniformly mixing components 14 to 17 at 85°C,
C: uniformly mixing components 9 to 13 and treating the mix on a dispersing machine (Disper),
D: slowly adding B to A at 85°C, emulsifying, and then adding C.

The resulting W/O mascara spread lightly, was dry and non-sticky, and showed satisfactory pigment dispersion.

It is noted that the invention is not limited to the aforementioned embodiments. While the embodiments are merely exemplary, any embodiments having substantially the same construction as the technical concept set forth in the following claims and exerting equivalent functions and results are believed to be within the spirit and scope of the invention.

## Claims

1. An organopolysiloxane having the compositional formula (1):
[Chem. 1]
(R¹₂SiO_{2/2})ₐ(R¹R²SiO_{2/2})_{b}(R¹R³SiO_{2/2})_{c}(R¹R⁴SiO_{2/2})_{d}(R¹SiO_{3/2})ₑ(SiO_{4/2})_{f}(R¹₃SiO_{1/2})_{2+e+2f} (1)
wherein R¹ is independently a monovalent hydrocarbon group selected from C₁-C₅ alkyl groups, C₆-C₁₅ aryl groups, and C₇-C₁₅ aralkyl groups,
R² is a C₆-C₂₀ alkyl group,
R³ is a group having the formula (3) which is bonded to a silicon atom in the polysiloxane backbone via a linking group having the formula (2):
-Q-O- (2)
wherein Q is a C₂-C₂₀ divalent hydrocarbon group,
or a glycerin group consisting of 2 to 10 glycerol units selected from the formulae (3) to (9): 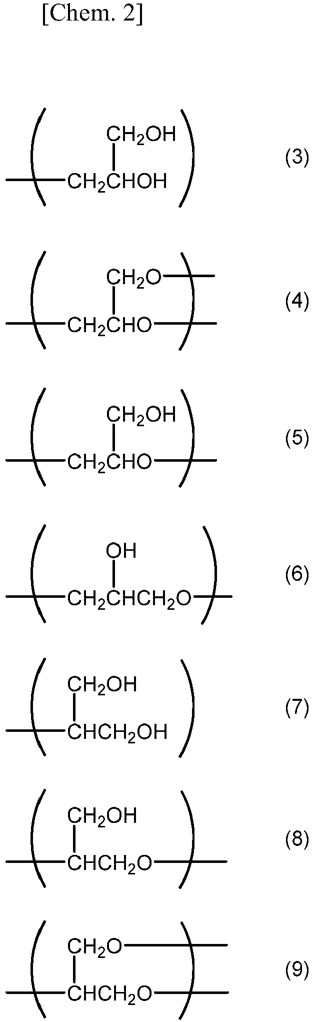
which is bonded to a silicon atom in the polysiloxane backbone via a linking group having the formula (2),
R⁴ is a group having the general formula (10), (11), (12) or (13):
[Chem. 3]
-(CH₂)₂-C_{g}H_{2g}-(SiOR¹₂)ₕ-SiR¹₃ (10)
-(CH₂)₂-C_{g}H_{2g}-SiR¹ₕ₁-(OSiR¹₃)₃₋ₕ₁ (11)
-(CH₂)₂-C_{g}H_{2g}-SiR¹ₕ₁-(OSiR¹ₕ₂(OSiR¹₃)₃₋ₕ₂)₃₋ₕ₁ (12)
-(CH₂)₂-C_{g}H_{2g}-SiR¹ₕ₁-(OSiR¹ₕ₂(OSiR¹ₕ₃(OSiR¹₃)₃₋ₕ₃)₃₋ₕ₂)₃₋ₕ₁ (13)
wherein R¹ is as defined above, g and h each are an integer in the range: 0 ≤ g ≤ 5 and 0 ≤ h ≤ 100, h1 to h3 each are an integer of 0 to 2,
a, b, c, d, e, and f each are a number in the range: 1 ≤ a ≤ 9, 3 < b ≤ 8, 0.4 ≤ c ≤ 3, 0.3 ≤ d ≤ 5, e ≥ 0, and f ≥ 0, 7 ≤ a+b+c+d ≤ 20, and 0.2 ≤ (b+c)/(a+d) ≤ 3,
the organopolysiloxane having a viscosity of 200 to 3,000 mPa·s as measured at 25°C by a B-type viscometer according to the method of JIS K 7117-1: 1999.

2. The organopolysiloxane of claim 1 wherein the total amount of R² is 20 to 40% by weight of one molecule, and the total amount of R³ is 5 to 20% by weight of one molecule.

3. The organopolysiloxane of claim 1 or 2, having a weight average molecular weight of 1,500 to 7,500 as measured by gel permeation chromatography versus polystyrene standards.

4. The organopolysiloxane of any one of claims 1 to 3 wherein R² in formula (1) is a C₁₂ alkyl group.

5. The organopolysiloxane of any one of claims 1 to 4 wherein R³ in formula (1) is selected from groups having the general formulae (14) to (20).

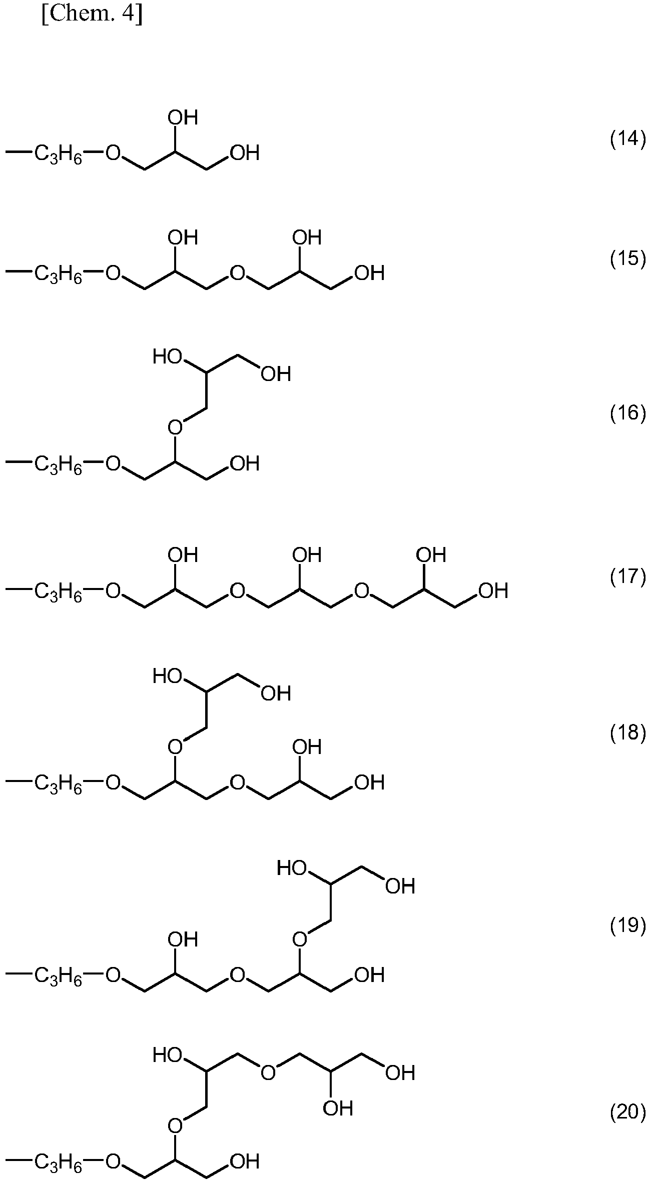

6. A cosmetic composition comprising (A) the organopolysiloxane of any one of claims 1 to 5.

7. The cosmetic composition of claim 6, further comprising (B) an oil-soluble UV absorber.

8. The cosmetic composition of claim 7 wherein the amount of component (B) is at least 4% by weight of the composition.

9. The cosmetic composition of any one of claims 6 to 8, further comprising (C) a powder.

10. The cosmetic composition of claim 9 wherein the amount of component (C) is at least 2% by weight of the composition.

11. The cosmetic composition of any one of claims 6 to 10, further comprising (D) an oil having a kinematic viscosity of 1 to 100 mm²/s at 25°C.
